# FASCICULE DE BREVET EUROPEEN

(11) **EP 0 939 626 B1**
(45) Date de publication et mention de la délivrance du brevet: **27.02.2002**
(21) Numéro de dépôt: 97938949.1
(22) Date de dépôt: 26.08.1997
(51) Int. Cl.: A61K 31/13, A61K 9/50, A61P 25/24

(54) **FORME GALENIQUE A LIBERATION PROLONGEE DE MILNACIPRAN**
MILNACIPRANHALTIGE DARREICHUNGSFORM MIT VERZÖGERTER WIRKSTOFFABGABE
GALENIC FORMULA WITH EXTENDED RELEASE OF MILNACIPRAN

(30) Priorité: 28.08.1996 FR 9610528
(43) Date de publication de la demande: 08.09.1999
(73) Titulaire: PIERRE FABRE MEDICAMENT, 92100 Boulogne-Billancourt (FR)
(72) Inventeur: PAILLARD, Bruno, F-63540 Romagnat (FR); GOUTAY, Eric, F-31650 Lauzerville (FR); AVAN, Jean-Louis, F-31290 Villefranche de Lauragais (FR); BOUGARET, Jo[l, F-31570 Lanta (FR)
(74) Mandataire: Ahner, Francis
(86) Numéro de dépôt international: FR9701525
(87) Numéro de publication internationale: WO9808495

(56) Documents cités:
- EP-A- 0 068 999
- EP-A- 0 200 638
- EP-A- 0 377 381
- EP-A- 0 687 472
- FR-A- 2 581 060

## Description

L'objet de la présente invention concerne la mise au point d'une forme multiparticulaire de type minigranules à libération contrôlée pour la voie orale, permettant l'administration en une prise journalière unique d'un antidépresseur particulier, à savoir : le Milnacipran.

L'originalité de cette invention repose sur la conception de cette forme minigranule ou minisphère qui, par association :
- de la concentration en Milnacipran par minigranule,
- des caractéristiques physico-chimiques du film d'enrobage,
- et de l'épaisseur de ce film,
permet de contrôler de façon surprenante la libération in vitro sur plusieurs heures d'une molécule dont la solubilité aqueuse est voisine de 800 g/l, rendant alors possible la monoprise de cette entité.

Le Milnacipran et ses énantiomères Cis se présentent sous forme de chlorhydrates, dont les solubilités aqueuses sont voisines de 800 g/l. Actuellement, ces molécules extrêmement solubles formulées avec du Phosphate dicalcique, de la Carboxyméthylcellulose calcique et de la Polyvinylpyrrolidone, conditionnées en gélules, ne permettent pas d'atteindre cet objectif, puisque la libération in vitro à partir de cette forme de 50 mg de Milnacipran est totale à trente minutes, obligeant alors la prise d'une gélule le matin et d'une gélule le soir.

Par opposition et de façon surprenante, la présente invention concerne une forme galénique à libération prolongée, destinée à l'administration par voie orale en une prise journalière unique de 60 à 240 mg de Milnacipran, se présentant sous une forme multiparticulaire réunissant une pluralité de minigranules comportant chacune une minisphère active comprenant un noyau de saccharose et/ou d'amidon de granulométrie comprise entre 200 et 2 000 µm et renfermant de 150 à 1 000 µg de Milnacipran ainsi qu'un agent liant, chaque minigranule étant enrobé par un film, à base d'au moins un polymère non hydrosoluble mais perméable aux liquides physiologiques, d'épaisseur comprise entre 20 et 100 µm, ladite forme galénique permettant une libération in vitro correspondant au profil suivant :
* entre 10 et 55 % de la dose libérée en 2 heures,
* entre 40 et 75 % de la dose libérée en 4 heures,
* entre 70 et 90 % de la dose libérée en 8 heures,
* entre 80 et 100 % de la dose libérée en 12 heures.

Cette forme minigranule dosée de 60 mg à 240 mg, plus précisément 120 mg de Milnacipran sous forme racémique, ou d'une dose thérapeutiquement équivalent de dérivé Cis Dextrogyre, assure le maintien de l'activité thérapeutique sur le nycthémère tout en écrêtant les valeurs des concentrations plasmatiques.

On rappellera tout d'abord que le Milnacipran, nouvel antidépresseur (brevets n° FR 2 508 035 - EP 200 638 et FR 2 640 972) présente une activité pharmacologique originale puisqu'il permet une inhibition mixte de la capture de la noradrénaline et de la sérotonine, sans effet sur la dopamine.

Sa structure chimique fait apparaître deux carbones asymétriques, conférant à la molécule une isomérie de type Cis et Trans, pour laquelle il a été démontré que les deux énantiomères Cis sont les formes actives, préférentiellement obtenue par synthèse.

Il a été également démontré que pour ces dérivés Cis, la forme dextrogyre est plus active que la forme levogyre.

C'est pourquoi l'objet de la présente invention s'applique à la fois à la molécule racémique le Milnacipran, mais également aux deux énantiomères Cis, puisque les propriétés physicochimiques impliquées dans les processus diffusionnels à partir de la forme restent identiques.

Le Milnacipran et ses formes énantiomères Cis présentent une biodisponibilité absolue supérieure à 85 % et une demi-vie biologique comprise entre 7 et 9 heures, ces propriétés étant tout à fait compatibles, au plan pharmacocinétique, avec la conception d'une forme à libération prolongée à une prise par jour.

De façon générale, deux étapes distinctes sont à considérer dans la réalisation technique des minigranules à libération prolongée, à savoir :
- la phase de réalisation des minisphères actives,
- la phase de pelliculage des minisphères actives.

Plusieurs technologies peuvent être utilisées pour la réalisation des minisphères actives :
* **le montage en turbine** qui consiste en un saupoudrage du principe actif à l'aide d'un liant sur des noyaux de saccharose ou de saccharose et d'amidon appelés encore non-pareils ;
* **le montage en lit d'air fluidisé** qui consiste à pulvériser une solution ou une dispersion de principe actif à l'aide d'un liant sur le lit de non-pareils. Cette pulvérisation peut se faire de haut en bas, de bas en haut (méthode du Würster) ou tangentiellement (méthode du rotor). Dans ce dernier cas, le principe actif peut être pulvérisé sous forme solide de façon concomitante au liquide de mouillage, à l'aide d'une trémie d'alimentation ;
* **la rotogranulation** qui permet d'obtenir des grains sphériques à partir d'un mélange actif-excipients adéquate sur lequel est pulvérisée une solution liante. Cette technologie est réalisable à partir de rotogranulateurs associés ou non à un lit d'air fluidisé ;
* **l'extrusion-sphéronisation** qui permet la réalisation de grains sphériques. Il s'agit d'obtenir, à partir d'un mélange actif-excipients adéquate, une masse plastique après mélange avec une solution liante.
   La masse plastique est alors extrudée à partir de différents systèmes permettant d'acheminer et/ou d'extruder cette masse (extrudeur à cylindres, extrudeur à engrenanges, à pistons, à mono ou bivis, à extrusion axiale ou radiale).
   Les extrudats obtenus sont alors sphéronisés dans un sphéroniseur adapté.

De manière classique, deux techniques sont employées pour l'obtention des minigranules enrobés :
- **la turbine** : les minisphères actives non enrobées sont introduites au sein d'une turbine perforée ou non. Une solution ou dispersion d'enrobage est alors pulvérisée sur le lit de minisphères à l'aide d'un pistolet ou buse ou de tout autre système adapté permettant la réalisation d'un film continu, régulier et reproductible ;
- **le lit d'air fluidisé** : en fonction du mode de pulvérisation choisi, le pelliculage des minisphères actives peut être réalisé en "topspray", en "bottom-spray" ou en "tangential-spray". Les deux dernières techniques assurent un enrobage plus régulier, plus continu et plus reproductible que la première technique.

Le problème majeur auquel est confronté l'homme de l'art pour la mise au point d'une forme à libération prolongée, de manière générale et de type minigranules en particulier, est la solubilité aqueuse de la molécule.

Dans le cas présent, le Milnacipran ainsi que ses énantiomères actifs sont très facilement solubles dans l'eau (solubilité = 800 g/l). Rappelons qu'il est impossible d'utiliser les formes bases pour des raisons de stabilité.
En effet, il est connu pour l'homme de l'art qu'avec de telles molécules, le formulateur devant mettre au point une forme à libération prolongée, est confronté au problème antinomique suivant :
* éviter le phénomène de brusque décharge très fréquent avec ce type de molécules et qui, dans certain cas, est synonyme d'effets secondaires,
* et assurer un parfait contrôle de la libération de la totalité de la dose administrée afin d'éviter tout perte de produit.

Certains formulateurs ont résolu ce problème :
* en associant au sein d'une même formulation plusieurs fractions de minigranules (DE-3 941 703), fraction non enrobée assurant la libération au cours des premières minutes, fraction enrobée avec un taux de polymère important contrôlant la diffusion durant les heures suivantes,
* ou en associant au sein d'une même formulation, des minigranules avec des pelliculages multi-couches (US-4,894,240 - WO-9 3097 67) ou des compositions chimiques de polymères d'enrobage de natures hétérogène (EP-508 653 - EP-0 322 277).

La présente invention apporte par la conception de cette forme minigranules à libération prolongée de Milnacipran, de solubilité aqueuse égale à 800 g/l, une solution moins contraignante pour le développeur.

En effet, les formules, objets de la présente invention, permettent par leur conception (teneur en principe actif par minigranule, épaisseur du film et composition du film) d'aboutir à une libération in vitro compatible avec l'objectif thérapeutique, tout en ne faisant appel qu'à un seul type de minigranules par formule.

Il est très difficile par absence de références de décrire, pour une molécule de solubilité supérieure à 500 g/l, ce que pourait être la conception d'une forme minigranule fabriquée par la technologie d'air fluidisé et/ou turbine.

A titre de comparaison, on se référera à la publication "Chlorpheniramine maleate controlled release spheres, I - Effect of ethylcellulose and dibutyl sebaçate levels", A.R. Oritz LABRADOR, E.S. CHALY (# 1146), Proceed. Intern. Symp. Control. Rel. Bioact. Mater., 20, 1993 où les auteurs appliquent jusqu'à 30 % d'Ethylcellulose associé au Dibutyl sébaçate pour obtenir un profil de libération retardée.

Dans le brevet EP-350 246, une base organique est utilisée comme agent modulateur de la libération.

Dans le brevet WO-953 491, ce sont des bases minérales qui sont utilisées comme supports contrôlant la libération.

Dans le document WO-9 201 446, on utilise des substances hydrophobes (parrafine, cires, alcool stéarilique) associées à un film de nature acrylique, pour contrôler la libération de principes actifs solubles tels que le salbutamol, la chlorpheniramine maleate.

Il est évident pour l'homme de l'art que ce type de formulation est très difficilement réalisable et transposable en lit d'air fluidisé.

Dans le brevet EP-249 949, le formulateur réalise un prémélange de la molécule soluble avec un polymère non ionique et une charge minérale, avant de les pulvériser sur le non pareil, sur lequel ensuite il applique le film non soluble. Dans cet exemple, il y a en fait une couche intermédiaire limitant la diffusion du principe actif vers le film d'enrobage.

La solution proposée dans le cadre de la présente invention est beaucoup plus simple et plus facilement industrialisable, comme le montrent plus particulièrement les exemples n° 13, 14 et 15 ci-après, car elle propose une forme minigranules composée d'un seul type de sphéroïdes :
- dont le noyau non pelliculé est de taille avantageusement comprise entre 710 et 850 µm,
- dont la teneur en principe actif par minigranule est de préférence voisine de 510 µg,
- et dont le pelliculage à base d'éthylcellulose en solution éthanolique est avantageusement compris entre 4 % et 12,5 %, soit une épaisseur faible de 20 µm à 80 µm par minigranule.

D'autres caractéristiques et avantages de la présente invention seront même compris à la lecture de la description détaillée faite ci-après, notamment en regard d'un certain nombre d'exemples de mise en oeuvre particulièrs.

Les composants utilisables pour le montage du Milnacipran en turbine sont :
* **des minisphères** composées de saccharose et/ou d'amidon, appelées encore non-pareils. Ces non-pareils ont été obtenus par dépôts successifs de saccharose et/ou d'amidon sur un cristal de saccharose. Les non-pareils utilisés sont composés de préférence de 75 % de saccharose et de 25 % d'amidon.
   Les non-pareils présentent différentes tailles allant de 200 microns à 2 mm.
   Les non-pareils utilisés présentent, de préférence, une taille comprise entre 500 et 1 000 microns et plus particulièrement comprise en 710 et 850 microns.
* **un agent liant** pulvérisé en solution sur le lit de minisphères. Cet agent liant peut être un dérivé de la cellulose tels que l'hydroxypropylméthyl cellulose, la méthylcellulose, l'hydroxypropylcellulose, la carboxyméthyl cellulose, peut être de la gélatine, peut-être une solution de saccharose, peut-être une gomme telle que la gomme arabique, la gomme adragante, la gomme guar, des pectines, des alginates. Il peut être dérivé de la polyvidone telle que la polyvinylpyrrolidone de différents poids moléculaires.
   Le taux de ces liants dans la solution de mouillage peut atteindre 3 à 50 % et dans le produit fini de 0,5 à 30 %.
   Le liant utilisé est, de préférence, la polyvinylpyrrolidone de poids moléculaire voisin de 50 000 Da (type K30) à un taux de 20 % dans la solution de mouillage et à un taux de 2 à 4 % dans les minisphères actives.
* **le solvant** utilisé pour véhiculer le liant peut être un solvant organique de type chlorure de méthylène, acétone, un alcool tel que l'isopropanol ou l'éthanol, de l'eau purifiée, ou des combinaisons miscibles de ces différents solvant. Le solvant utilisé de préférence est l'éthanol.
* **un agent** destiné à éviter l'agglomération des minigranules entre eux est de façon avantageuse utilisé par saupoudrage sur le lit de noyaux.
   Des dérivés de la silice, des oxydes métalliques tel que l'oxyde de titane, des silicates tel que le talc peuvent être utilisés à des taux compris entre 0,5 et 20 % du poids des minisphères actives. Le talc est utilisé de préférence à un taux compris entre 3 et 4 %.
   Le Milnacipran est saupoudré sur le lit de noyaux et son taux peut être compris entre 5 et 90 % dans les minisphères. Ce taux est compris de préférence entre 45 et 55 % et est de préférence de 51 %. Soit une concentration de Milnacipran par minigranules comprise entre 450 µg et 550 µg et préférentiellement voisine de 510 µg puisque la minisphère active pèse de préférence 1 mg.

Les composants utilisés lors du montage du Milnacipran en lit d'air fluidisé sont :
* **des minisphères** composées de saccharose et/ou d'amidon appelées encore non-pareils. Ces non-pareils ont été obtenus par dépôts successifs de saccharose et/ou d'amidon sur un cristal de saccharose. Les non-pareils utilisés sont composés de préférence de 75 % de saccharose et de 25 % d'amidon.
   Les non-pareils présentent différentes tailles allant de 200 µm à 2 mm.
   Les non-pareils utilisés présentent, de préférence, une taille comprise entre 500 et 1 000 µm et plus particulièrement entre 710 et 850 µm.
* **Un agent liant** pulvérisé en solution sur le lit de minisphères. Cet agent liant peut être un dérivé de la cellulose tels que l'hydroxypropylméthyl cellulose, la méthylcellulose, l'hydroxypropylcellulose, la carboxyméthyl cellulose, peut être de la gélatine, peut être une solution de saccharose, peut être une gomme telle que la gomme arabique, la gomme adragante, la gomme guar, des pectines, des alginates. Il peut être dérivé de la polyvidone telle que la polyvinylpyrrolidone de différents poids moléculaires.
   Le taux de ces liants dans la solution de mouillage peut atteindre 3 à 50 % et dans le produit fini de 0,5 à 30 %.
   Le liant utilisé est de préférence la polyvinylpyrrolidone de poids moléculaire voisin de 50 000 Da (type K30) à un taux de 0 à 20% et plus précisément 6,7 % dans la solution de mouillage et de 5 à 25% plus précisément 15,4 % dans les minisphères actives.
* **Le solvant** utilisé pour véhiculer le liant peut être l'isopropanol et/ou l'acétone, l'éthanol et/ou l'acétone et est de préférence l'isopropanol.
* **Le Milnacipran** est pulvérisé sous forme d'une dispersion à un taux de 10 à 40 % dans le solvant. Son taux préférentiel est de 20 % dans le solvant.
   Le Milnacipran peut représenter 5 à 90 % de la minisphère active. Il est compris de préférence entre 40 et 50 % et est de préférence de 46%.
   Soit une concentration de Milnacipran par minigranule comprise entre 150 et 185 µg et préférentiellement voisine de 170 µg quand les non-pareils sont de taille comprise entre 500 et 600 µm.
   Soit une concentration de Milnacipran par minigranule comprise entre 440 et 555 µg et préférentiellement voisine de 510 µg quand les nonpareils sont de taille comprise entre 710 et 850 µm.
   Soit une concentration de Milnacipran par minigranule comprise entre 680 et 850 µg et préférentiellement voisine de 780 µg quand les nonpareils sont de taille comprise entre 850 à 1 000 µm.

Les composants utilisés lors de la réalisation de minisphères actives en extrusion-sphéronisation sont :
* **Un diluant** qui peut être de nature hydrophile ou hydrophobe.
   Le diluant hydrophile composants peut être de nature cellulosique telles que la cellulose microcristalline, la cellulose sodique voire l'hydroxypropylméthylcellulose. Le lactose et l'amidon peuvent également être utilisés.
   Le diluant lipophile peut être un monoglycéride, un diglycéride, un triglycéride.
   Ces diluants représentent 5 à 90 % de la minisphère active. Le diluant utilisé de préférence est la cellulose microcristalline à un taux compris entre 25 et 75 % et de préférence à un taux de 50 %.
* **Un agent liant.** Cet agent liant peut être un dérivé de la cellulose telles que l'hydroxypropylméthylcellulose, la méthylcellulose, l'hydroxypropyl cellulose, la carboxyméthylcellulose, peut être de la gélatine, peut être une solution de saccharose, peut être une gomme telles que la gomme arabique, la gomme adragante, la gomme guar, peut être des pectines ou des alginates. Il peut être dérivé de la polyvidone telle que la polyvinylpyrrolidone de différents poids moléculaires.
   Le taux de ces liants dans la solution de mouillage peut atteindre 0 à 50%.
   Cet agent liant peut varier de 0 à 20 % dans les minisphères actives.
   Lorsque le diluant est la cellulose microcristalline, il n'est pas nécessaire d'utiliser d'agent liant.
* **Un solvant** est utilisé pour mouiller le mélange des différents composants et en faire une masse extrudable.
   Des alcools tels que l'éthanol ou l'isopropanol peuvent être utilisés en association ou pas avec de l'eau purifiée. L'eau purifiée est utilisé de préférence pour mouiller les particules.
* **Le principe actif,** lors de cette opération, est introduit à un taux compris entre 5 et 90 % du poids des minisphères actives, de préférence à un taux compris entre 25 et 75% du poids des minisphères actives et plus particulièrement voisin de 50 %. Soit une concentration de Milnacipran par minigranule comprise de préférence entre 250 et 750 µm et plus particulièrement de 500 µg.

Les composants utilisés lors de la réalisation des minisphères actives en rotogranulation sont:
* Un diluant qui peut être de nature hydrophile ou hydrophobe.
   Le diluant hydrophile composants peut être de nature cellulosique telles que la cellulose microcristalline, la cellulose sodique voire
   l'hydroxypropylméthylcellulose. Le lactose et l'amidon peuvent également être utilisés.
   Le diluant lipophile peut être un monoglycéride, un diglycéride, un triglycéride.
   Ces diluants représentent 5 à 90 % de la minisphère active. Le diluant utilisé de préférence est la cellulose microcristalline à un taux compris entre 40 et 60 % et de préférence à un taux de 50 %.
* **Un agent liant**. Cet agent liant peut être un dérivé de la cellulose telles que l'hydroxypropylméthylcellulose, la méthylcellulose, l'hydroxypropyl cellulose, la carboxyméthylcellulose, peut être de la gélatine, peut être une solution de saccharose, peut être une gomme telles que la gomme arabique, la gomme adragante, la gomme guar, peut être des pectines ou des alginates. Il peut être dérivé de la polyvidone telle que la polyvinylpyrrolidone de différents poids moléculaires.
   Le taux de ces liants dans la solution de mouillage peut atteindre 0 à 50%.
   Cet agent liant peut varier de 0 à 20 % dans les minisphères actives.
   Lorsque le diluant est la cellulose microcristalline, il n'est pas nécessaire d'utiliser d'agent liant.
* **Un solvant** est utilisé pour mouiller le mélange des différents composants et en faire une masse extrudable.
   Des alcools tels que l'éthanol ou l'isopropanol peuvent être utilisés en association ou pas avec de l'eau purifiée. L'eau purifiée est utilisée de préférence pour mouiller les particules.
* **Le principe actif,** lors de cette opération, est introduit à un taux compris entre 5 et 90 % du poids des minisphères actives, de préférence à un taux compris entre 40 et 60 % du poids des minisphères actives et plus particulièrement voisin de 50 %. Soit une concentration de Milnacipran par minigranule comprise de préférence entre 500 et 750 µm et plus particulièrement de 625 µg.

L'enrobage des minisphères se compose de polymères filmogènes insolubles dans l'eau mais perméables aux liquides physiologiques et qui laissent passer, par des phénomènes de diffusion, le Milnacipran en solution.
* Les agents d'enrobage utilisés traditionnellement sont des dérivés de copolymères acryliques, des alkylcelluloses, l'éthylcellulose et les laques d'origine naturelle telle que la gomme shellac.

Dans l'invention sont utilisés des copolymères méthacryliques de type poly (éthylacrylate, méthyl méthacrylate) en dispersion aqueuse commercialisés sous le nom d'Eudragit NE30D, ou de type poly (éthyl acrylate, méthyl méthacrylate, chlorure de triméthyl ammoniuméthyl méthacrylate chloride) en solvants organiques (RS100 ou RL100) ou en dispersion aqueuse RS30D/RL30D dont la perméabilité est fonction du taux de groupements ammonium (RL>RS).

Les polymères sont utilisés à des taux compris entre 5 et 50 % en poids de polymère sec par rapport au poids des minisphères actives.

Le polymère d'appellation commerciale Eudragit RL100 ou RL30D n'est pas utilisé à ces taux mais respectivement en association avec les polymères d'appellation commerciale Eudragit RS100 ou RS30D à des taux pouvant aller de 1 à 20 % du taux global de polymères d'enrobage.
* D'autres agents d'enrobage tels que l'éthylcellulose de différents grades sont utilisés. Dans l'invention, l'éthylcellulose peut être utilisée en solution dans un solvant tels que le dichlorométhane, l'acétate d'éthyle, le méthanol et l'éthanol ou un mélange de ces solvants. L'éthanol est utilisé de préférence.

Le taux d'éthylcellulose représente, en poids de polymère sec, 2,5 à 50 % du poids des minisphères actives et, de préférence, est compris entre 3 et 15 % et plus particulièrement entre 4 et 12,5 %.

L'éthylcellulose peut également se présenter sous forme de dispersion aqueuse prête à l'emploi tels que l'Aquacoat ECD30 ou le Surelease qui se différencie entre autres de l'Aquacoat ECD30 par le fait que le plastifiant est intégré à la dispersion.
* Les dispersions aqueuses d'éthylcellulose sont utilisées à des taux de substances solides représentant de 5 à 30 % du poids des minisphères actives. Les taux utilisés représentent de préférence 12,5 à 17,5 % de ces minisphères.
* Il est possible d'associer à ces dispersions des hydroxypropyl méthylcelluloses de bas poids moléculaire solubles dans l'eau, de la polyvinylpyrrolidone, des polyéthylène glycols de bas poids moléculaire ou toutes autres substances solubles susceptibles de favoriser la création de pores dans la membrane à des taux compris entre 1 et 20 % du film d'enrobage.
   Dans notre cas, il n'a pas été intégré de polymère soluble à la formule.
* Les polymères d'enrobage utilisés dans cette étude ont été associés à des plastifiants destinés à améliorer la formation et la qualité du film.
   Les plastifiants utilisés peuvent être: le diméthylphtalate, le diéthylphtalate, le dibutylphtalate, le dibutylsébaçate, le triéthylcitrate, l'acétyltriéthylcitrate, le tributylcitrate, l'acétyltributylcitrate, la triacétine, les polyéthylènes glycols, le propylène glycol, les glycerols, les glycérides tels que le monoglycéride acétylé, l'huile de coco fractionnée et l'huile de ricin.
   Les plastifiants sont utilisés à des taux compris entre 0 et 50 % du poids de polymère sec.
   Ils sont utilisés de préférence à des taux compris entre 15 et 25 % du poids de polymère sec.
   Le plastifiant utilisé de préférence est le triéthylcitrate à un taux représentant 20% du poids de polymère sec.
* Des agents de charge sont utilisés tels que des oxydes métalliques, des silices, des silicates en particulier le silicate de magnésium à des taux compris entre 10 et 100 % du poids de polymère sec.
   Les taux utilisés avec les dérivés acryliques sont compris de préférence entre 50 et 100 % de talc alors qu'ils sont compris entre 20 et 70 % avec l'éthylcellulose en solution éthanolique et de préférence égale à 50 %.
* Des agents antimoussants, tels les dérivés de la silicone, peuvent être intégrés aux différentes formulations à des taux compris entre 0 et 0,5 % des minisphères actives.

L'invention pourra être mieux comprise à l'aide des exemples non limitatifs qui suivent, qui constituent des modes de mise en oeuvre particuliers des différents procédés et qui font référence à différents types de composants selon la formule.

Les exemples allant de 1 à 5 décrivent les procédés et composants utilisés pour la réalisation des minigranules actifs.

Les exemples allant de 6 à 16 décrivent les procédés et composants utilisés pour la réalisation de l'enrobage des minigranules actifs.

La technique de contrôle de dissolution in vitro utilisée pour caractériser ces formules, est directement inspirée de la monographie USP XXIII, DISSOLUTION, avec l'appareil à palette (type 2), la vitesse des palettes est fixée à 120 RPM, le milieu de dissolution est soit le tampon phosphate (0,066M) pH=7,2, soit de l'eau purifiée.

### Exemple n° 1 :

L'exemple 1 décrit la réalisation de minigranules dosés en Milnacipran à partir d'une fixation du principe actif en turbine.

Dans une turbine pleine de taille laboratoire, un kilogramme de non pareils de taille comprise entre 710 et 850 microns est introduit. Une solution éthanolique de polyvinylpyrrolidone K30 à 20 % est pulvérisée afin d'humecter les neutres et les rendre légèrement collants.

Le Milnacipran, après tamisage sur tamis d'ouverture de maille de 600 microns, est projeté à l'aide d'une pelle sur le lit de noyaux. Du talc peut être introduit au lit de noyau en cas de collage. Le cycle se termine après un court séchage qui permet au Milnacipran d'adhérer complètement au noyau.

Les cycles se succèdent ainsi jusqu'à épuisement du Milnacipran.

En fin de fixation, une proportion de talc est déposée en surface du minigranule à l'aide de la solution de PVP K30 afin d'isoler le Milnacipran de l'humidité extérieure.

Un séchage terminal en turbine est effectué sur une période de quatre heures venant s'ajouter aux séchages intermédiaires réalisés au terme de chaque cycle.

Cette opération de fixation a été réalisée sur une turbine de taille pilote et semi-industrielle. Le mode opératoire reste le même si ce n'est que les quantités de matières premières sont multipliées par le facteur 8 et 56.

| Composants | Quantités mises en oeuvre (kg) | | | Formule centésimale (%) |
|---|---|---|---|---|
| | Turbine laboratoire | Turbine pilote | Turbine semi-ind. | |
| Non-pareils | 0,832 | 6,658 | 46,60 | 42,62 |
| Milnacipran | 1,000 | 8,000 | 56,00 | 51,22 |
| PVP K30 | 0,048 | 0,382 | 2,70 ± (80 %) | 2,45 |
| Talc | 0,072 | 0,580 | 4,06 | 3,71 |

Au cours de cette opération de montage, la concentration par minigranule non enrobé est voisine de 510 µg, conduisant pour une dose administrable de 100 mg, à une composition renfermant environ 195 minigranules.

### Exemple n° 2 :

Le procédé utilisé dans cet exemple est celui du lit d'air fluidisé équipé d'un système de pulvérisation par le bas de type Würster. L'appareillage utilisé est de marque GLATT de type GPCG1.

Une solution d'Isopropanol concentrée à 6,7 % de PVP K30 est réalisée.
Le Milnacipran est ajouté à cette solution par dispersion à l'aide d'un disperseur Ultra Turrax pendant dix minutes. Le Milnacipran représente 20,2 % de la dispersion.

La dispersion est maintenue sous agitation douce durant le procédé (agitateur à hélices).

Dans la cuve à matière de type Würster, un kilogramme de neutres, dont la taille peut être comprise entre 500 et 600 µm, 710 et 850 µm ou 850 et 1000 µm, est introduit.

La température moyenne d'entrée d'air est de 65° C, le débit de pulvérisation moyen est de 20 g/min, le débit d'air de 85 m³/h, la pression de pulvérisation de 2 bars et le diamètre de la buse de pulvérisation de 1 mm.

Une fois la dispersion de Milnacipran épuisée, les minigranules sont séchés durant 5 minutes en lit d'air fluidisé à 65° C puis en étuve ventilée à 45° C pendant 24 heures.

Le mode opératoire décrit ci-dessus est transposé au niveau d'un appareillage pilote de marque GLATT de type GPCG5.

La préparation de la dispersion du Milnacipran dans la solution isopropanolique de PVP K30 se fait dans les mêmes conditions que précédemment en multipliant les quantités mises en oeuvre par le facteur 8,4.

Dans la cuve à matière de type Würster, 8,4 Kg de neutres dont la taille est comprise entre 710 et 850 microns sont introduits.

La température moyenne d'entrée d'air est de 55° C, le débit de pulvérisation moyen de 150 g/min, le débit d'air de 260 m³/h, la pression d'atomisation de 3,5 bars et le diamètre de la buse est de 1,2 mm.

Une fois la dispersion de Milnacipran épuisée, les minigranules sont séchés durant 5 minutes en lit d'air fluidisé à 55° C puis en étuve ventilée à 45° C pendant 24 heures.

**Description des formules :**

| **Composants** | **Quantités mises en oeuvre (kg)** | | **Formule centésimales (%)** |
|---|---|---|---|
| | GPCG1 | GPCG5 | |
| Non-pareils | 1,00 | 8,40 | 38,46 |
| PVP K30 | 0,40 | 3,36 | 15,38 |
| Milnacipran | 1,20 | 10,08 | 46,15 |

Au cours de cette opération de montage, la concentration par minigranule est voisine de 510 µg conduisant, en fonction de la taille de la gélule (tailles n° 1 à n° 00, à une dose administrable de 60 à 240 mg.

### Exemple n° 3 :

Le Milnacipran et un excipient, la cellulose microcristalline dont le nom de marque est l'Avicel PH101, sont mélangés à partie égale (150 grammes chacun) dans un mélangeur de type Turbula T2 pendant 10 minutes.

Le mélange ainsi constitué est humidifié dans un mélangeur planétaire de type Kenwood Major.

Le liquide utilisé est l'eau purifiée et sa quantité est ajustée afin d'obtenir une masse plastique. Le temps de mouillage dure 5 minutes et le malaxage 3 minutes.

La masse plastique ainsi obtenue est extrudée dans un extrudeur de type GABLER PHARMEX 35T à l'aide d'une vis de 450 mm de long pour 45 mm de diamètre qui tourne à la vitesse de 50 RPM.

La grille d'extrusion présente des mailles de 1 mm et est orientée dans l'axe de la vis sans fin.

L'extrusion se fait à température ambiante.

Les extrudats obtenus sont ensuite sphéronisés à l'aide d'un sphéroniseur GABLER SPHAEROMAT SPH 250 dont la vitesse est réglée à 1000 RPM durant 1 minute puis à 120 RPM durant 9 minutes.

Le séchage se fait en étuve ventilée à la température de 40° C jusqu'à ce que le taux d'humidité résiduelle soit inférieur à 3 %.

Les minigranules ainsi obtenus présentent une granulométrie comprise entre 1 mm et 1,4 mm et un rapport d'élongation de 0,86.

**Description de la formule :**

| **Composants** | **Quantités mises en oeuvre (g)** | **Formule centésimale (%)** |
|---|---|---|
| Milnacipran | 150 | 50 |
| Avicel PH101 | 150 | 50 |

Au cours de cette opération de montage, la concentration par minigranule est voisine de 500 µg conduisant, en fonction de la taille de la gélule (tailles n° 1 à n° 00, à une dose administrable de 60 à 240 mg.

### Exemple n° 4 :

Le procédé opératoire utilisé dans cet exemple est également l'extrusion-sphéronisation. Mais l'extrusion, à l'inverse de l'exemple n° 3, se fait radialement à la vis.

Le mélange des différents composants qui varient en qualité et en quantité en fonction des deux formules décrites, s'effectue dans un granulateur LODIGE® pendant 5 minutes à vitesse constante.

Le liquide de mouillage est de l'eau purifiée qui représente en fonction des formules de 15 à 18 % de la masse.

La masse plastique est extrudée radialement et forcée à vitesse constante à travers une grille de 1 mm de diamètre.

Les extrudats obtenus sont sphéronisés par lot de 500 g dans un sphéroniseur Colette Marumerizer durant 5 à 10 minutes en fonction des formules.

Les minigranules obtenus sont séchés 24 heures à 40° C en étuve ventilée.

**Description des formules** :

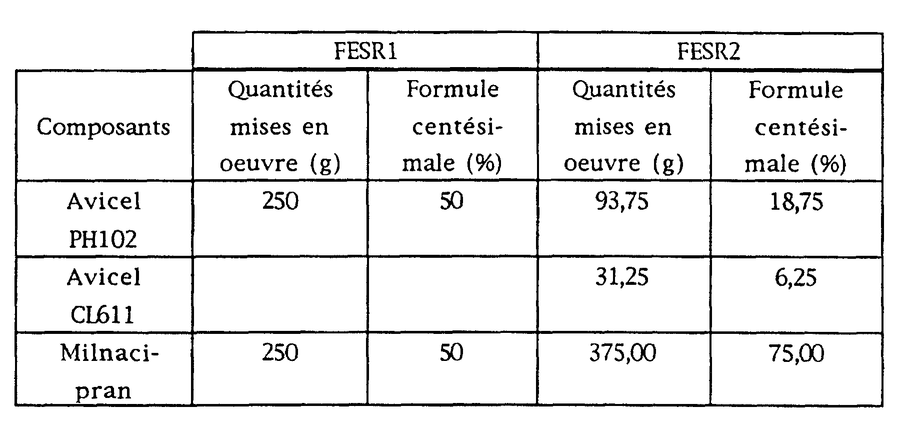

Au cours de cette opération de montage, la concentration par minigranule est de 400 µg ou de 1160 µg conduisanten fonction de la taille de la gélule (tailles n° 1 à n° 00), à une dose administrable de 60 à 240 mg.

### Exemple n° 5 :

Dans l'exemple n° 5, la réalisation des minigranules dosés en Milnacipran se fait par granulation sphérique en lit d'air fluidisé.

L'appareillage se caractérise par un disque rotatif dont l'écartement avec la paroi de la cuve à matière permet de moduler l'admission d'air. Sous l'action combinée de la rotation du disque et du flux d'air ascendant, les particules suivent un mouvement en spiral. Dans le même temps, un liquide de mouillage est pulvérisé tangentiellement au mélange. L'association de ces opérations conduit à l'obtention de particules sphériques.

Le mélange composé de 250 g de Milnacipran et de 250 g d'Avicel PH101 s'effectue au sein de la cuve à matière pendant trois minutes. L'air est admis à un débit de 100 m³/h et le disque rotatif tourne à 180 RPM.

Au sein du même appareillage, l'humidification de la masse s'effectue par pulvérisation tangentielle d'eau purifiée.

Le débit de pulvérisation est de à 20 g/min et la vitesse de rotation du disque est augmentée de 180 RPM à 1080 RPM par incrémentation de 180 RPM toutes les minutes.

La température d'entrée d'air est de 50° C, la pression d'atomisation est de 2 bars et le diamètre de la buse de 1,2 mm.

Durant l'humidification du mélange, les particules s'agglomèrent, donnant des grains qui, par roulement sur les parois lisses et cylindriques de la cuve, se densifient en prenant un aspect sphérique.

Une fois les minigranules obtenus, ceux-ci sont séchés au sein de la cuve rotor pendant 10 minutes à 60 C puis pendant 12 heures à 40° C en étuve ventilée.

Ces minigranules se caractérisent par une répartition granulométrique comprise entre 700 et 1400 microns et un facteur d'élongation de 0,85.

**Description de la formule** :

| **Composants** | **Quantité mise en oeuvre (g)** | **Formule centésimale (%)** |
|---|---|---|
| Avicel PH101 | 250 | 50 |
| Milnacipran | 250 | 50 |

Au cours de cette opération de montage la concentration par minigranule est voisine de 625 µg, conduisant en fonction de la taille de la gélule (tailles n° 1 à n° 00), à une dose administrable de 60 à 240 mg.

Les exemples allant de 6 à 16 décrivent les procédés et compsants utilisés pour la réalisation de l'enrobage des minigranules.

### Exemple n° 6 :

Des minigranules réalisés par la méthode décrite dans l'exemple n° 2 sont enrobés à l'aide du même procédé technologique.

Dans 1 500 g d'une solution organique composée de 80 % d'Isopropanol et de 20 % d'acétone, sont dissous 180 g de polymère filmogène dont le nom commercial est l'Eudragit RS 100.

A cette solution est ajouté 27 g de diéthylphtalate qui joue le rôle de plastifiant.

Dans 750 g d'une solution organique de même composition que celle préalablement décrite, sont ajoutés 90 g de talc jouant le rôle d'agent antiadhérent. Cette proportion de talc est dispersée par un polydisperseur de type ultra-turrax.

Cette dispersion est incorporée à la solution d'Eudragit RS100 et maintenue sous agitation (agitateurs à hélices) durant la pulvérisation. La préparation d'enrobage est maintenue sous agitation (agitateur à hélices) durant la pulvérisation.

La température moyenne d'air d'entrée est de 45° C, le débit moyen de pulvérisation est de 10 g/min, le débit d'air de 85 m³/h, la pression de pulvérisation de 1,5 bars et le diamètre de la buse de 0,8 mm.

En fonction de la quantité de liquide d'enrobage déposée sur les minigranules, le taux de polymère sec d'enrobage peut représenter 20 %, 25% ou 30% du poids des minigranules actives.

**Description des formules :**

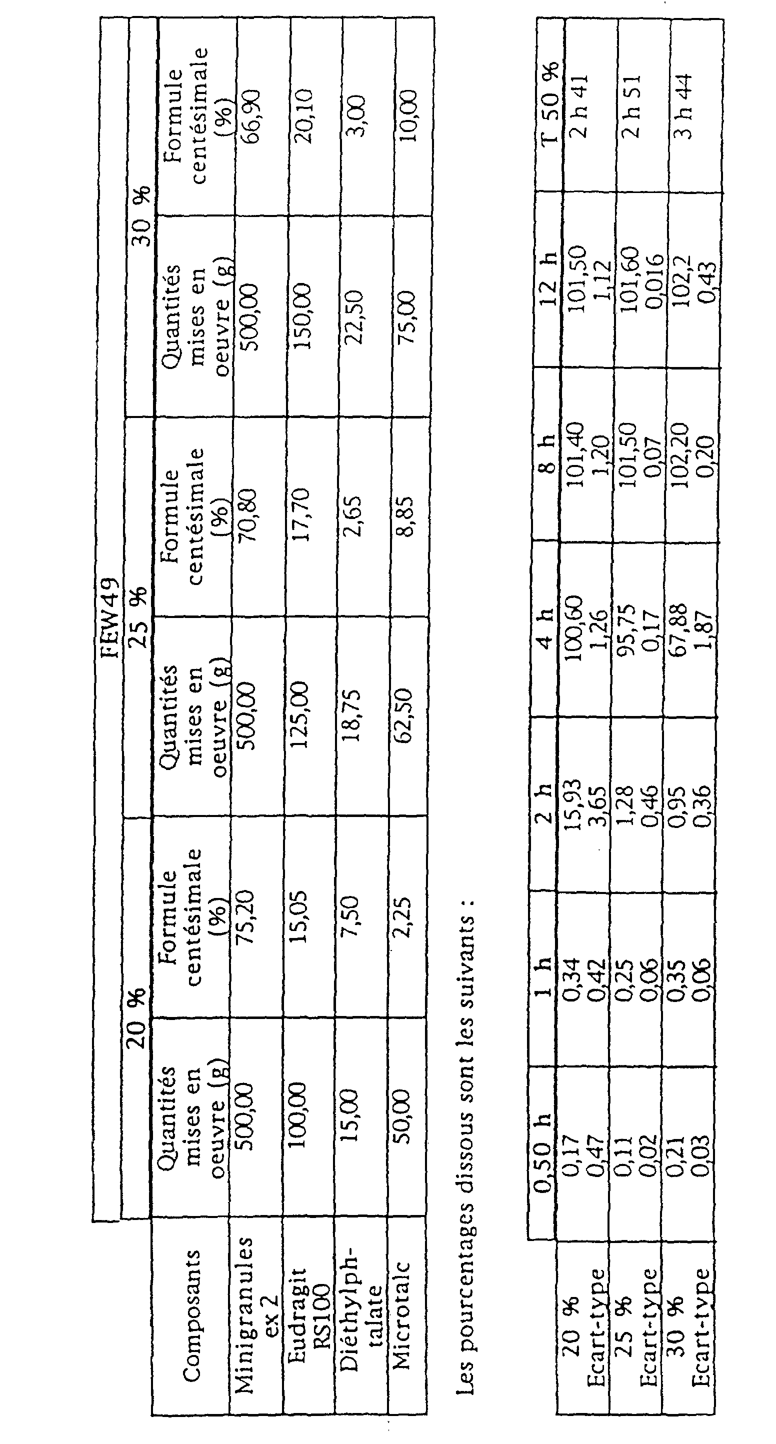

Ces formules, dont la concentration de Milnacipran par minigranule non enrobé est de 510 µg et dont les épaisseurs de film sont égales à 112, 141 et 169 µm (masses surfaciques égales à 4,74, 5,93 et 7,12 mg/cm²), ne permettent pas d'atteindre l'objectif in vitro précédemment cité.

### Exemple n° 7 :

Cet exemple se différencie principalement du précédent par le fait que le polymère filmogène utilisé se présente sous forme de dispersion aqueuse dont le nom commercial est Eudragit RS30D, que le plastifiant est le triéthylcitrate et que les nonpareils ont une taille comprise entre 850 et 1000 microns.

Dans 417 g de dispersion aqueuse d'Eudragit RS30D, sont incorporés 19 g de triéthylcitrate sous agitation douce. Dans 400 g d'eau purifiée, sont introduits 50 g de talc dispersés à l'aide d'un polydisperseur de type ultra-turrax. Une fois obtenue une dispersion homogène de talc, celle-ci est ajoutée à la première préparation et maintenue sous agitation douce pendant toute la durée de la pulvérisation.

La température d'air d'entrée est comprise entre 50 et 55° C, le débit moyen de pulvérisation est de 10 g/min, le débit d'air est de 85 m³/h, la pression de pulvérisation de 2 bars et le diamètre de la buse de 0,8 mm.

En fonction de la quantité de liquide d'enrobage déposée sur les minigranules, le taux de polymères d'enrobage peut représenter 20 ou 25 % du poids de minigranules non enrobés.

**Description des formules :**

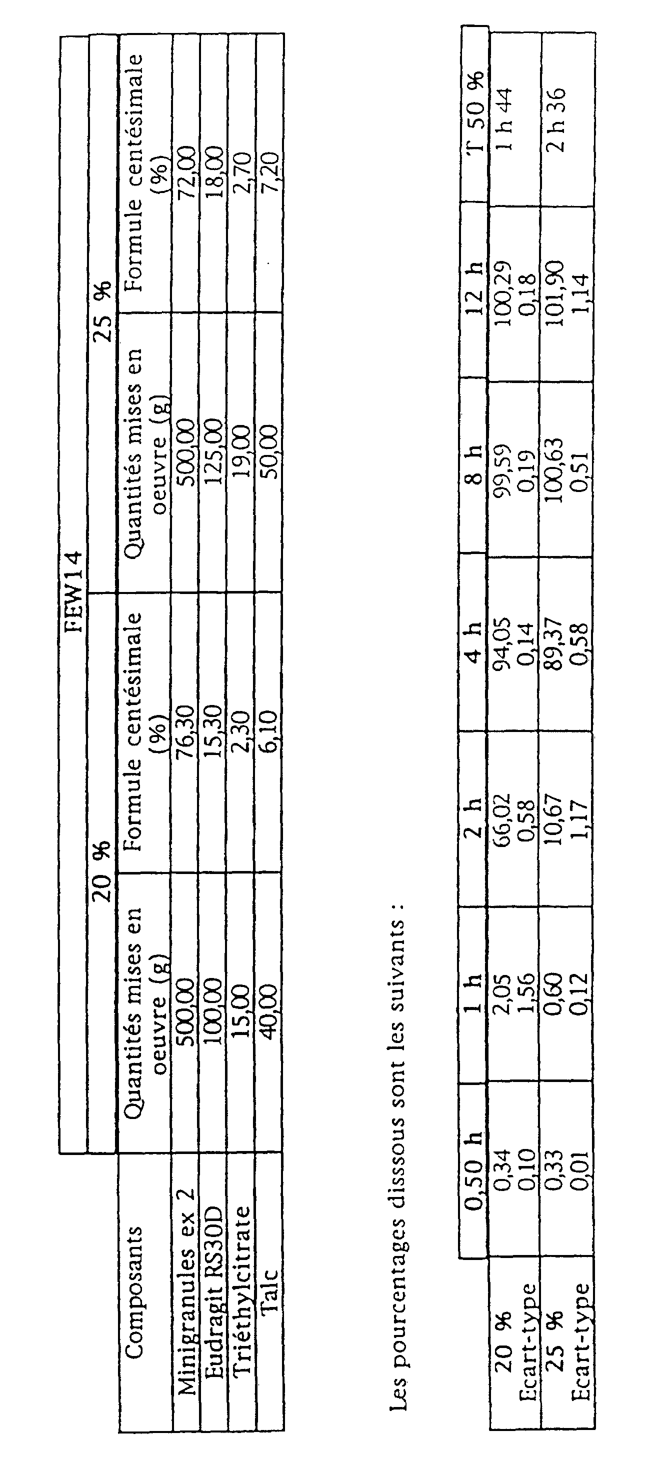

Ces formules, dont la concentration de Milnacipran par minigranule non enrobé est de 780 µg et dont les épaisseurs de film sont égales à 151 et 188 µm (masses surfaciques égales à 5,5 et 6,85 mg/cm²), ne permettent pas d'atteindre l'objectif in vitro précédemment cité.

### Exemple n° 8 :

Cet exemple se différencie du précédent par le fait que le polymère filmogène utilisé est de l'Eudragit NE30D et non plus de l'Eudragit RS30D, que l'on n'utilise pas de plastifiant car la température de transition vitreuse du polymère est faible et que l'on utilise 75 % de talc par rapport au poids de polymère sec et que les non-pareils ont une taille comprise entre 710 et 850 microns.

Dans 323,2 g d'eau purifiée sont dispersés, à l'aide d'un polydisperseur de type ultra-turrax, 65,6 g de talc.

Une fois obtenue une dispersion homogène, celle-ci est incorporée à 291,7 g d'Eudragit NE30D sous agitation douce (agitateur à hélices). La dispersion ainsi obtenue est maintenue sous agitation pendant la pulvérisation.

La température moyenne d'entrée d'air est de 35° C, le débit de pulvérisation moyen est de 10 g/min, le débit d'air moyen de 110 m³/h, la pression de pulvérisation de 2 bars et le diamètre de la buse de 0,8 mm.

En fonction de la quantité de liquide d'enrobage déposée sur les minigranules, le taux de polymère sec d'enrobage peut représenter 10, 12,5, 15, 17,5 % du poids de microgranules non enrobés.

**Description des formules :**

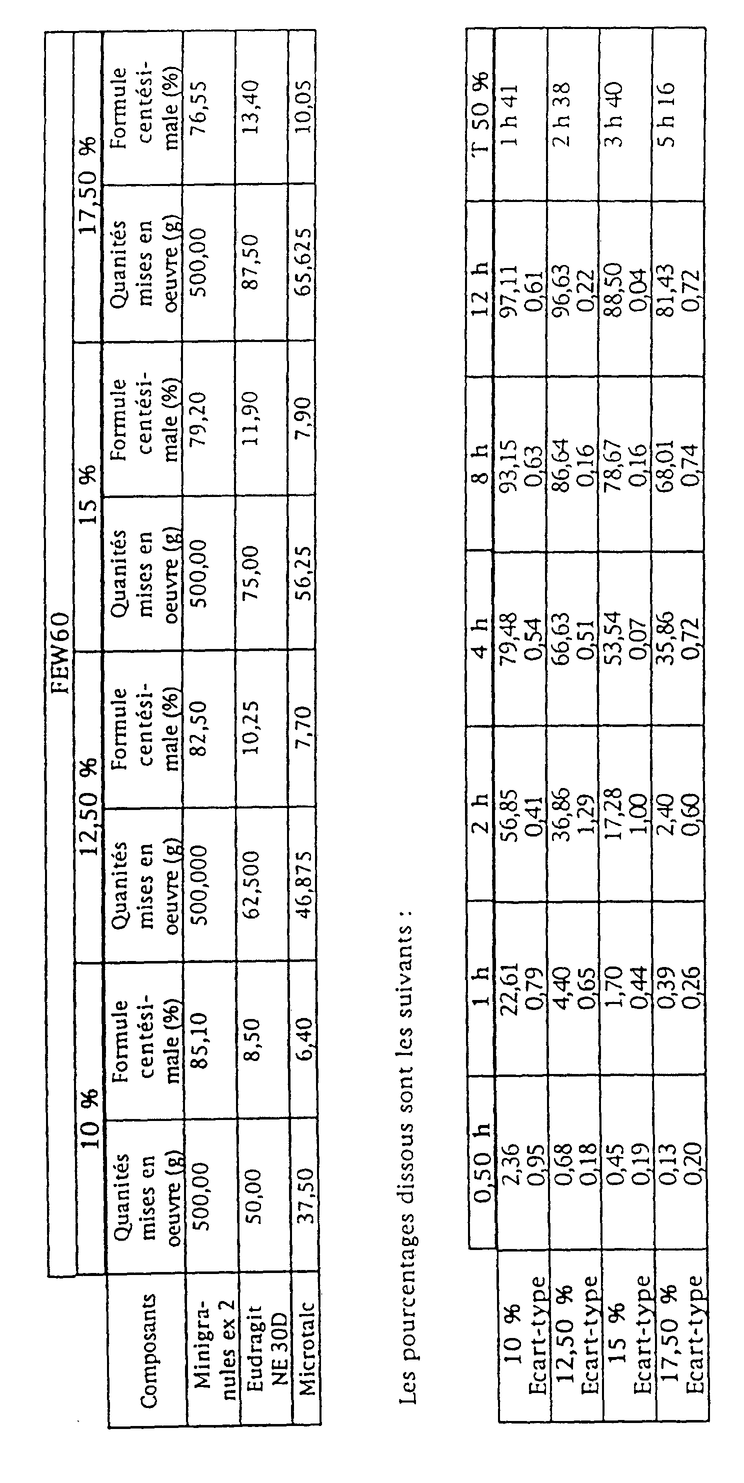

Les formules à 10, et 17,5 % de polymère dont la concentration de Milnacipran par minigranule non enrobé est de 510 µg et dont les épaisseurs de film sont égales à 51, et 89 µm (masses surfaciques égales à 2,15 et 3,94 mg/cm²), ne permettent pas d'atteindre l'objectif in vitro précédemment cité.

Par contre, de façon surprenante, la formule à 12,5 % et 15 % de polymère, renfermant une concentration de Milnacipran par minigranule non enrobé de 510 µg, dont l'épaisseur de film est voisine de 63 et 76 µm (masse surfacique égale à 2,81 et 3,37 mg/cm²) atteignent l'objectif in vitro évoqué auparavant.

Ces formules aux doses unitaires de 60 à 240 mg, et plus particulièrement de 100 mg et 150 mg de Milnacipran, peuvent être mises en gélule de tailles n° 1 ou n° 0+.

### Exemple n° 9 :

L'exemple n° 9 se différencie principalement des précédents exemples par le fait que le polymère d'enrobage n'est plus un copolymère des acides acryliques métha et éthacryliques mais est de l'éthylcellulose présenté sous forme de dispersion aqueuse à un taux compris entre 24,5 et 29,5 %. Cette dispersion est commercialisée sous le nom d'Aquacoat ECD30 et se compose également d'un tensio-actif, le lauryl sulfate de sodium, à un taux compris entre 0,9 et 1,7 % et d'un agent stabilisant, l'alcool céthylique, à un taux compris entre 1,7 et 3,3 %.

Dans 246 g d'eau purifiée, sont incorporés 334 g d'Aquacoat ECD sous agitation douce (agitateur à hélices) puis 20 g de plastifiant, le triéthylcitrate, sont ajoutés à la dispersion. Pour permettre une bonne répartition du plastifiant, l'agitation est poursuivie pendant 30 minutes avant le début de la pulvérisation durant laquelle elle est maintenue.

La température moyenne d'air d'entrée est de 52,5° C, le débit moyen de pulvérisation est de 10 g/min, le débit d'air est de 85 m³/h, la pression de pulvérisation est de 2 bars et le diamètre de la buse de 0,8 mm.

En fonction de la quantité de liquide d'enrobage déposée sur les microgranules, le taux de polymère sec d'enrobage peut représenter 10, 15 ou 20 % du poids de microgranules non enrobés.

A la fin de la pulvérisation, un séchage en lit d'air fluidisé est réalisé à 40° C pendant 5 minutes. Les minigranules sont ensuite déchargés et séchés pendant 24 heures à 40° C en étuve ventilée.

En fonction de la quantité de liquide d'enrobage déposée sur les microgranules, le taux de polymère sec d'enrobage peut représenter 10, 15 ou 20 % du poids de minigranules non enrobés.

**Description des formules :**

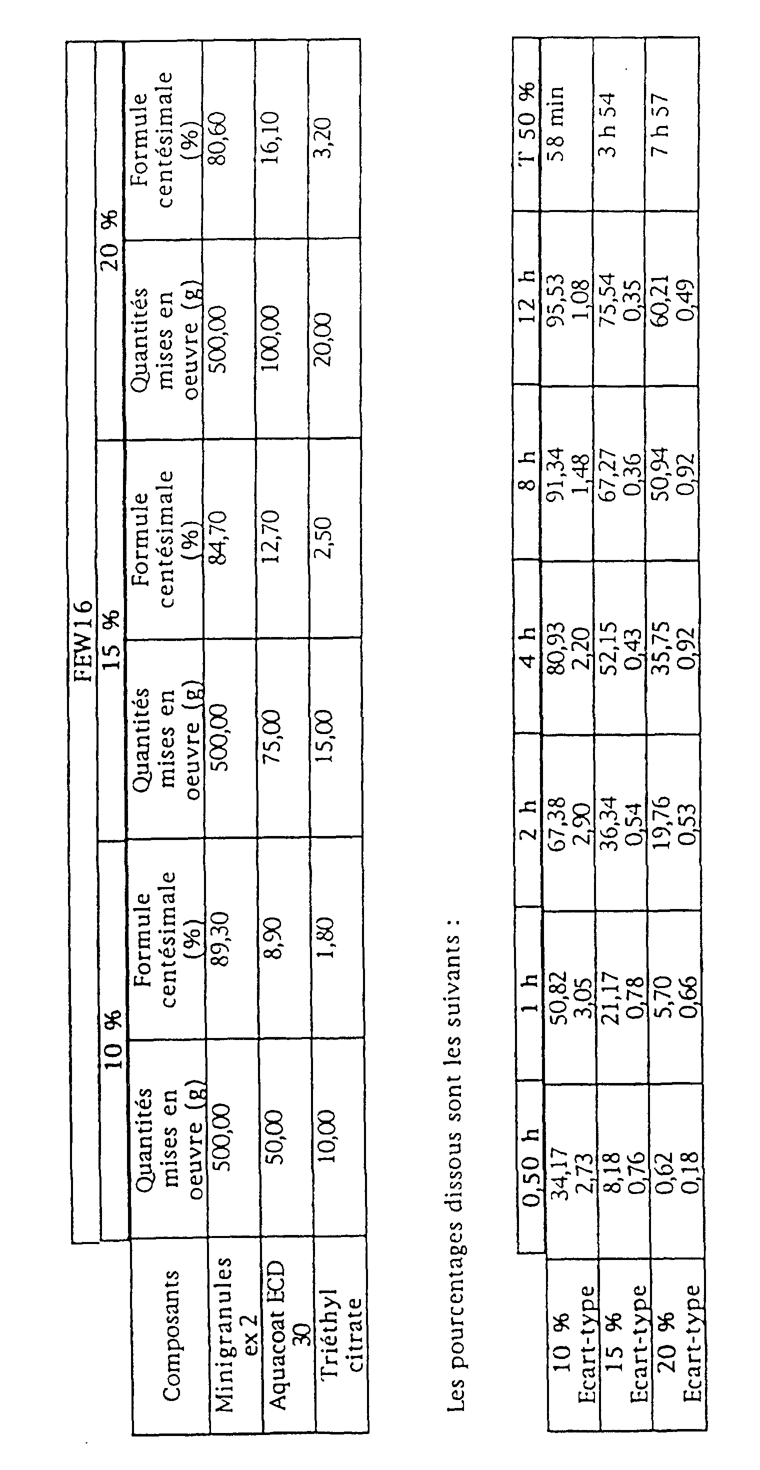

Ces formules, dont la concentration de Milnacipran par minigranule est de 510 µg et dont les épaisseurs de film sont égales à 75, 113 et 150 µm (masses surfaciques égales à 2,74, 4,11 et 5,48 mg/cm²), ne permettent pas d'atteindre l'objectif in vitro précédemment cité.

### Exemple n° 10 :

Cet exemple se différencie de l'exemple précédent par le fait que la dispersion aqueuse d'éthylcellulose est commercialisée sous le nom de Surelease et que sa composition est différente de l'Aquacoat ECD30.

Cette dispersion se compose d'éthylcellulose, d'un plastifiant qui peut être le dibutyl sébaçate ou de l'huile de coco fractionnée appelée encore miglyol, d'un stabilisant coplastifiant l'acide oléïque et d'une base aqueuse d'hydroxyde d'ammonium. Dans cet exemple précis, le plastifiant est du dibutyl sébaçate.

La préparation de la dispersion d'enrobage se fait en incorporant à 915 grammes d'eau purifiée, 400 g de Surelease E-7-7050 sous agitation douce (agitateur à hélices).

En fonction de la quantité de liquide d'enrobage déposée sur les microgranules, le taux de polymère sec d'enrobage peut représenter 10, 15 ou 20 % du poids des microgranules non enrobés.

L'opération d'enrobage ainsi que le séchage des minigranules s'effectuent de la même façon que dans l'exemple 10.

**Description des formules :**

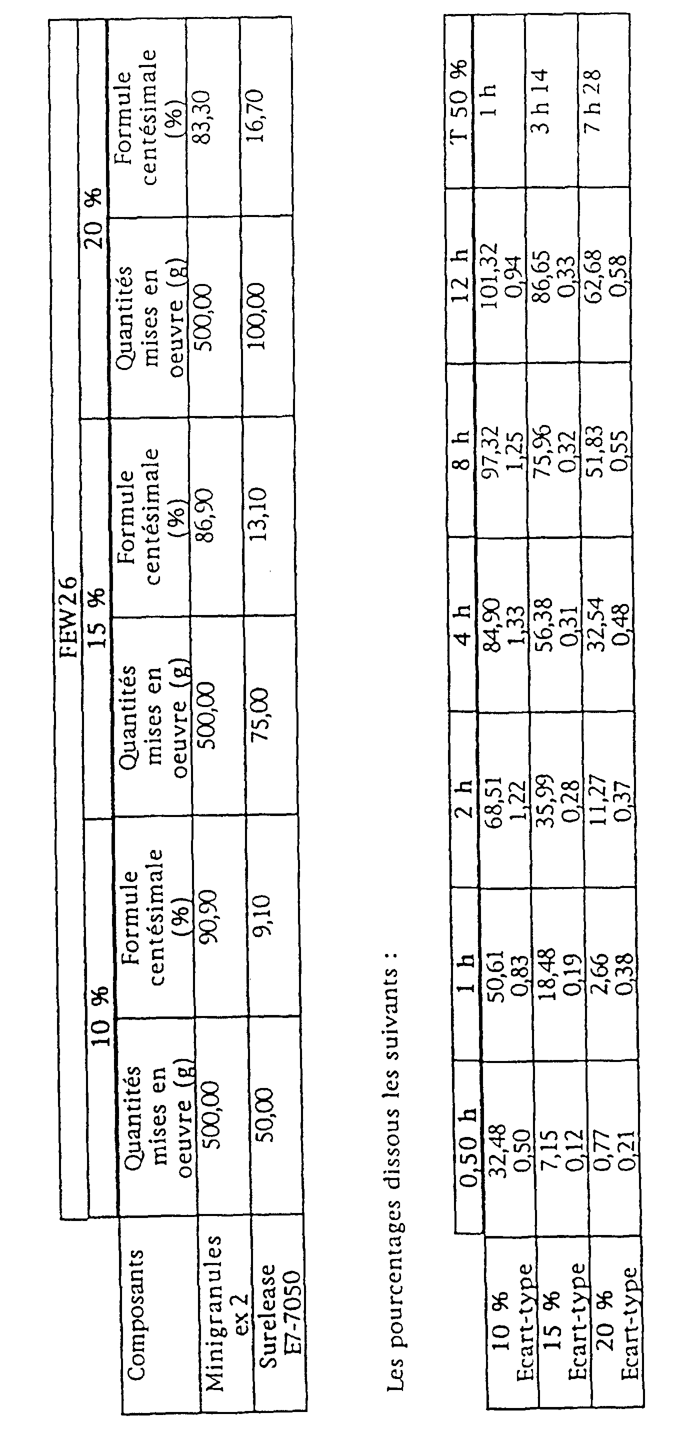

Les formules à 10 et 20 % de polymère dont la concentration de Milnacipran par minigranule non enrobé est de 510 µg, dont les épaisseurs de film sont égales à 52 et 103 µm (masses surfaciques égales à 2,27 et 4,54 mg/cm²) ne permettent pas d'atteindre l'objectif in vitro précédemment cité.

Par contre, de façon surprenante, la formule à 15 % de polymère, renfermant une concentration de Milnacipran par minigranule non enrobé de 510 µg, dont l'épaisseur de film est égale à 77,5 µm (masse surfacique égale à 3,41 mg/cm²) atteint l'objectif in vitro évoqué auparavant.

Ces formules aux doses unitaires de 60 à 240 mg, et plus particulièrement de 110 mg et 220 mg de Milnacipran, peuvent être mises en gélule de tailles n° 1 ou n° 00.

### Exemple n° 11 :

Cet exemple se différencie des exemples n° 9 et 10 par le fait que le polymère d'enrobage, I'éthylcellulose, ne se présente plus sous la forme d'une dispersion mais sous la forme d'une solution éthanolique du polymère filmogène.

Dans 900 grammes d'éthanol, sont incorporés progressivement 50 g d'éthylcellulose sous agitation vive (agitateur à hélices). Le mélange est maintenu sous agitation pendant une heure.

Dans 300 g d'éthanol, sont dispersés, à l'aide d'un polydisperseur de type Ultra-turrax, 25 grammes de talc durant le temps nécessaire à l'homogénéisation de la dispersion.

A la solution éthanolique d'éthylcellulose, sont ajoutés 10 g de plastifiant, le dibutyl sébaçate et la dispersion de talc sous agitation douce (agitateur à hélices). Cette agitation est maintenue durant la pulvérisation.

Durant l'opération d'enrobage, la température moyenne d'entrée d'air est de 40° C, le débit moyen de pulvérisation est de 11 g/min, le débit d'air moyen de 85 m³/h, la pression de pulvérisation de 2 bars et le diamètre de la buse de 0,8 mm.

Les minigranules sont ensuite séchés en lit d'air fluidisé à 35° C pendant 5 minutes. Un séchage complémentaire en étuve ventilée pendant 24 heures à 40° C est opéré.

En fonction de la quantité du liquide d'enrobage déposé sur les microgranules, le taux de polymère sec d'enrobage peut représenter 5, 7,5, 10 % du poids de microgranules non enrobés.

**Description des formules :**

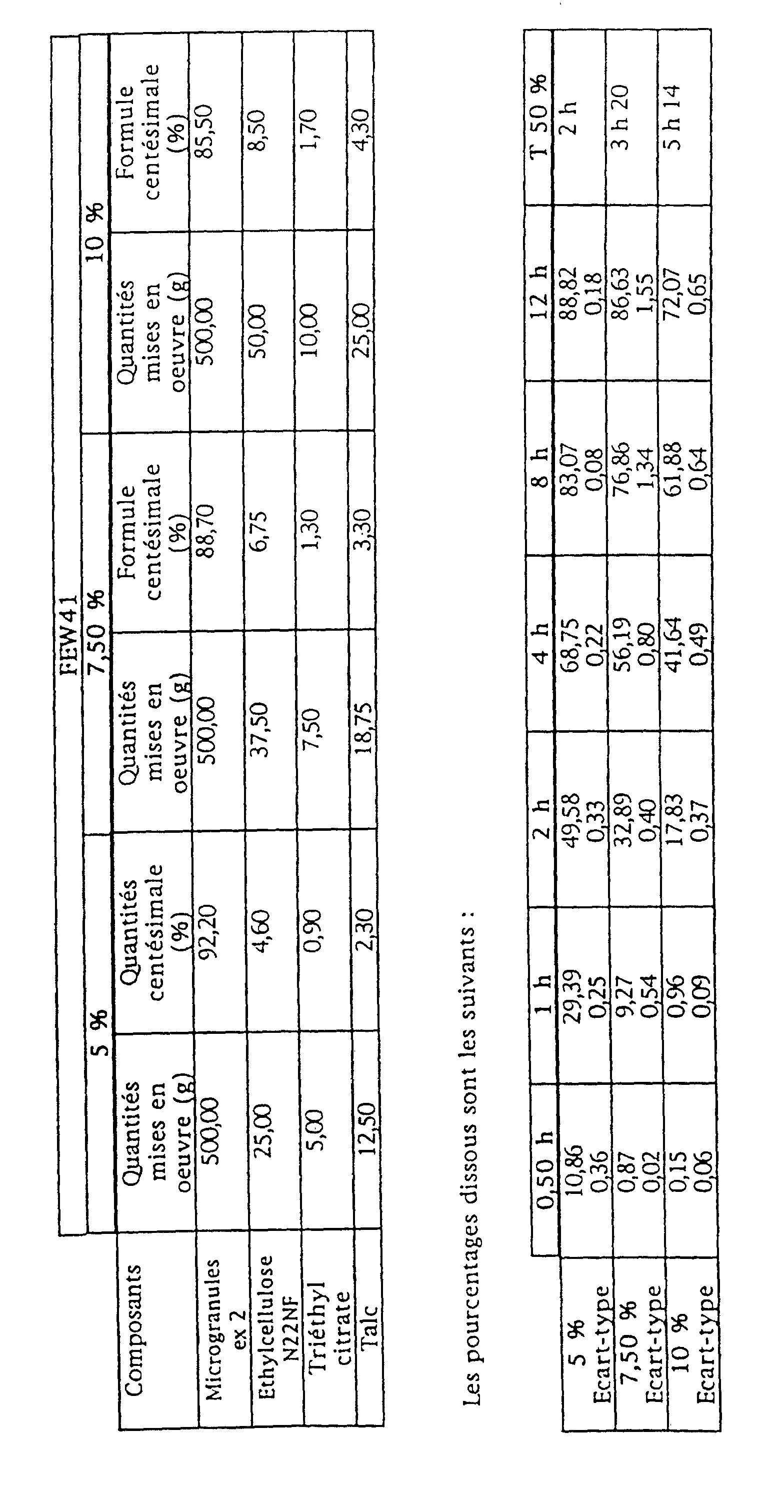

La formule à 10 % de polymère dont la concentration de Milnacipran par minigranule non enrobé est de 510 µg, dont l'épaisseur de film est de 54 µm (masse surfacique égale à 2,32 mg/cm²) ne permet pas d'atteindre l'objectif in vitro précédemment cité.

Par contre, de façon surprenante, les formules à 5 et 7,5 % de polymère renfermant une concentration de Milnacipran par minigranule non enrobé de 510 µg, dont les épaisseurs de film sont égales à 27 et 40 µm (masses surfaciques égales à 1,16 et 1,74 mg/cm²) atteignent l'objectif in vitro évoqué auparavant.

Ces formules aux doses unitaires de 60 à 240 mg, et plus particulièrement de 60 mg et 120 mg de Milnacipran, peuvent être mises en gélule de tailles n° 3 ou n° 1.

### Exemple n° 12 :

Cet exemple se différencie de l'exemple 11 par le fait que les non-pareils utilisés dans l'opération de fixation du F2207 en lit d'air fluidisé sont d'une taille comprise entre 500 et 600 microns et non pas comprise entre 710 et 850 microns et que le plastifiant est le dibutylsébaçate et non pas le triéthylcitrate.

En fonction de la quantité de liquide d'enrobage déposée sur les microgranules, le taux de polymère sec d'enrobage peut représenter 5, 7,5 ou 10 % du poids de microgranules non enrobés.

**Description des formules :**

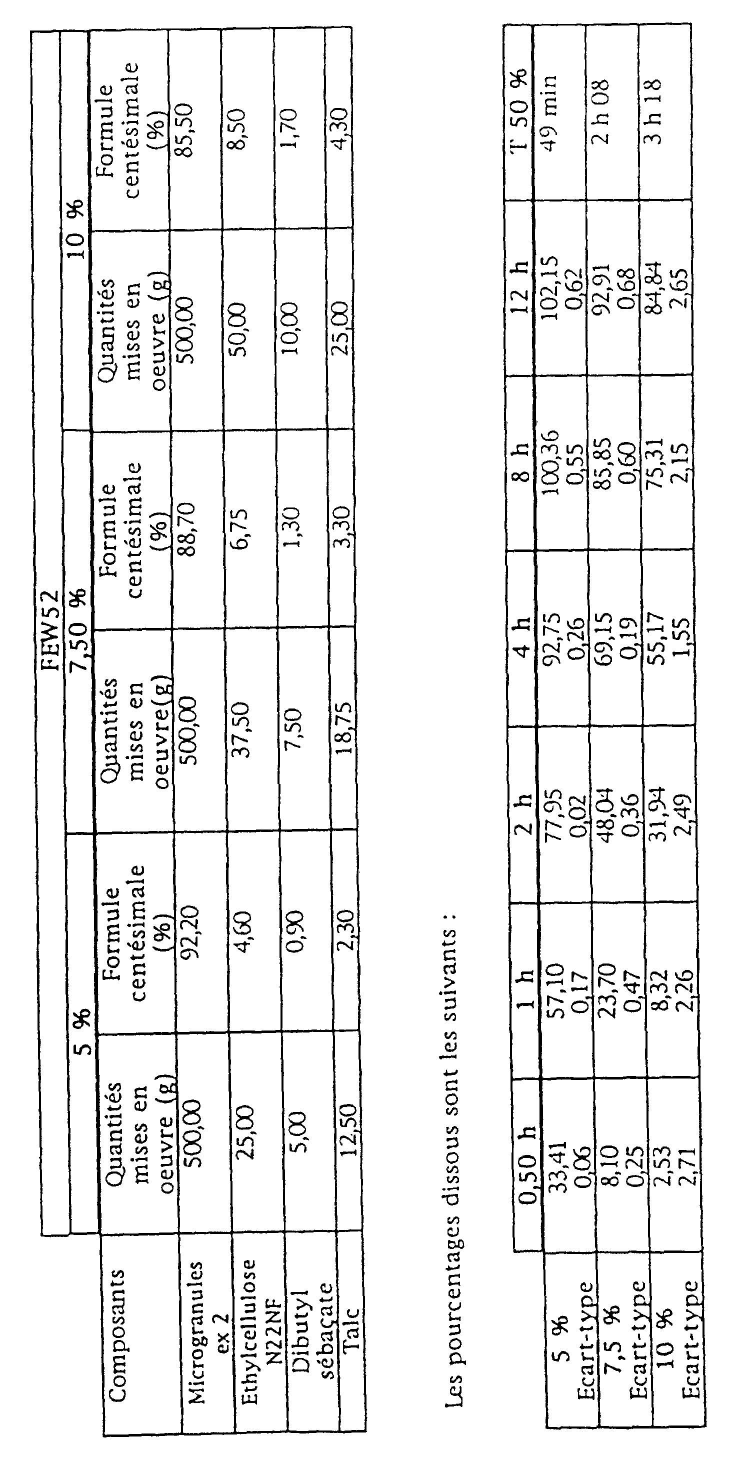

La formule à 5 % de polymère, dont la concentration de Milnacipran par minigranule est de 175 µg et dont l'épaisseur de film est égale à 12 µm (masse surfacique égale à 0,77 mg/cm²), ne permet pas d'atteindre l'objectif in vitro précédemment cité.

Par contre, de façon surprenante, les formules à 7,5 et 10 % de polymère, renfermant une concentration de Milnacipran par minigranule de 175 µg dont les épaisseurs de film sont égales à 18 et 28 µm (masses surfaciques égales à 1,15 et 1,54 mg/cm²) atteignent l'objectif in vitro évoqué auparavant.

Ces formules à la dose unitaire de 60 à 240 mg, et plus particulièrement de 120 mg de Milnacipran, peuvent être mises en gélule de tailles n° 1 ou n° 0+.

### Exemple n° 13 :

Cet exemple se différencie des exemples précédents par le fait que la fixation du Milnacipran s'est faite suivant l'exemple n° 1 et non plus suivant l'exemple n° 2. Par contre, la composition de l'enrobage est identique celle décrite dans l'exemple n° 11.

Les conditions opératoires sont modifiées au niveau de la température moyenne d'entrée d'air qui est fixée à 50° C.

En fonction de la quantité de liquide d'enrobage déposée sur les microgranules, le taux de polymère sec d'enrobage peut représenter 5, 7,5 et 10 % du poids de microgranules non enrobés.

**Description des formules :**

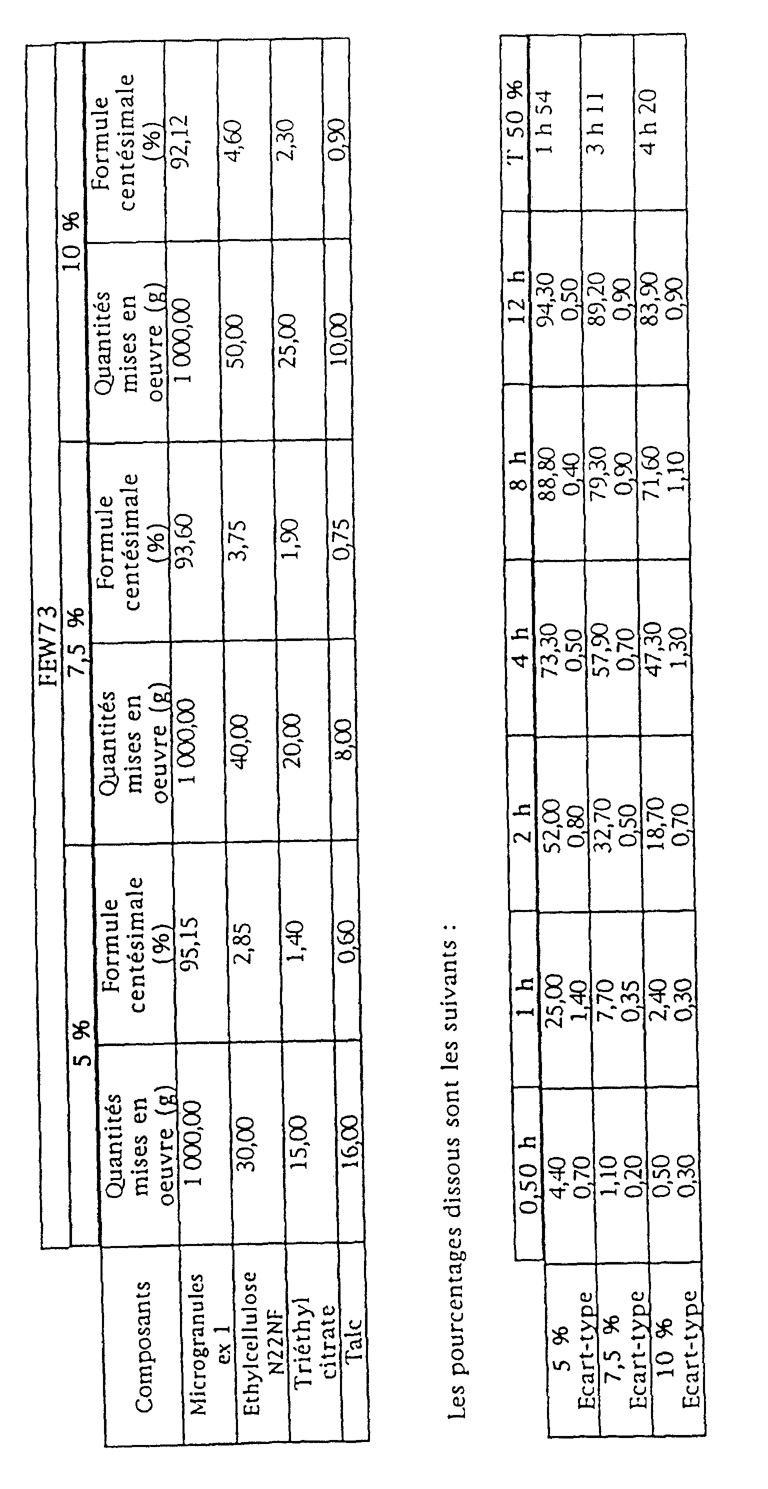

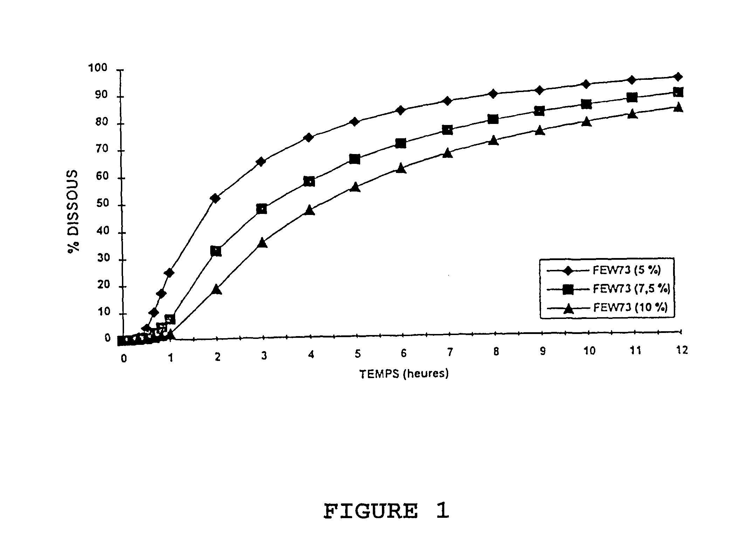

De façon surprenante, la formule FEW73 à 5, 7,5 et 10 % d'éthylcellulose, renfermant une concentration de Milnacipran par minigranule de 510 µg, dont les épaisseurs de film sont égales à 28, 43 et 57 µm (masses surfaciques égales à 1,19, 1,79 et 2,38 mg/cm²) atteignent l'objectif in vitro évoqué auparavant. Les courbes de cinétiques moyennes de dissolution dans le tampon phosphate à pH 7,2 sont indiquées ci-après dans la figure n° 1 annexée.

Ces formules aux doses unitaires de 60 à 240 mg, et plus particulièrement de 60 mg et 240 mg de Milnacipran, peuvent être mises ne gélule de tailles n° 3 ou n° 00.

### Exemple n° 14 :

Cet exemple se différencie du précédent par le fait que l'opération d'enrobage des minigranules se fait sur un appareillage pilote de type GLATT GPCG5.

Les proportions des différents composants sont multipliés par le facteur 15.

La température moyenne d'air d'entrée est fixée à 52°C, le débit moyen de pulvérisation est de 110 g/min, le débit d'air moyen est de 300 m³/h, la pression de pulvérisation est de 3 bars et le diamètre de la buse de 1,2 mm.

En fonction de la quantité de liquide d'enrobage déposée sur les minigranules, le taux de polymère sec d'enrobage peut représenter 5, 7,5 et 10% du poids de minigranules non enrobés.

**Description des formules :**

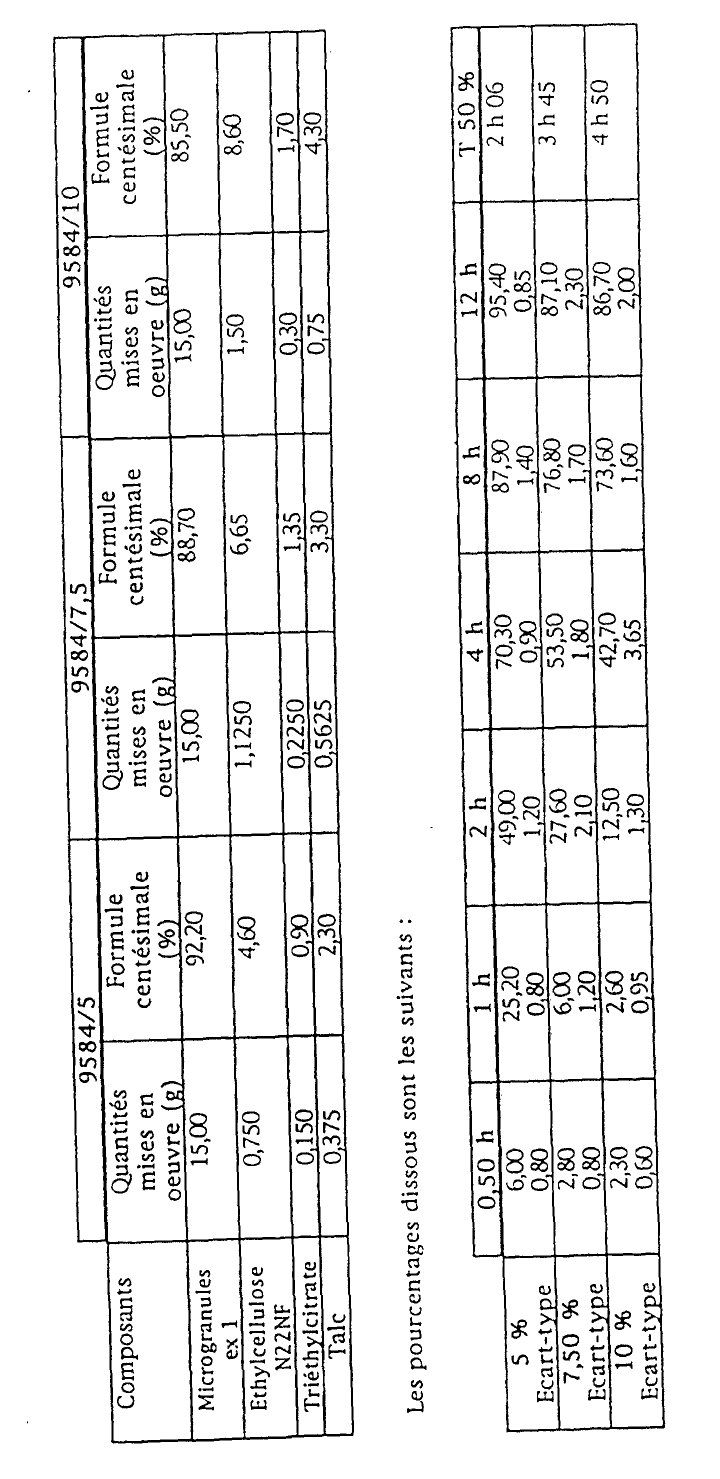

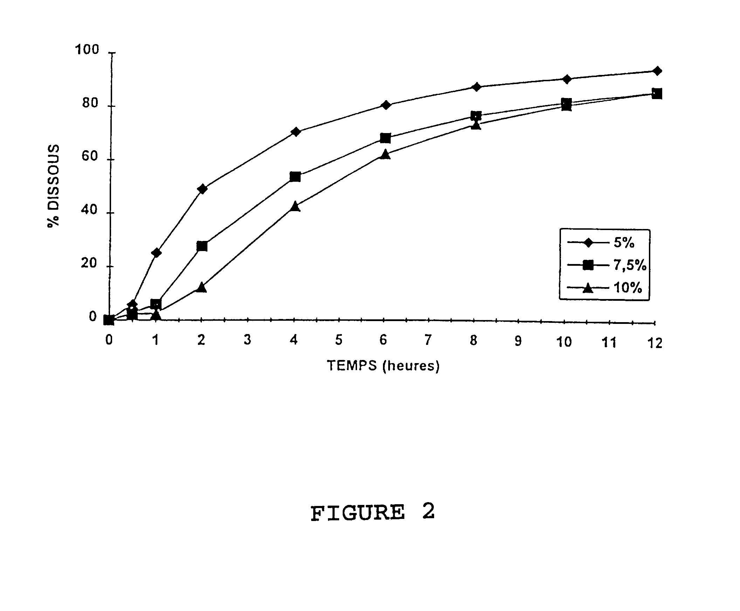

De façon surprenante, la formule 9584 à 5, 7,5 et 10 % d'éthylcellulose, renfermant une concentration de Milnacipran par minigranule non enrobé de 510 µg, dont les épaisseurs de film sont égales à 28, 43 et 57 µm (masses surfaciques égales à 1,19, 1,79 et 2,38 mg/cm²) atteignent l'objectif in vitro évoqué auparavant. Les courbes de dissolution moyennes in vitro dans le tampon phosphate à pH 7,2 sont données dans la figure 2 annexée.

Ces formules aux doses unitaires de 60 à 240 mg et plus particulièrement de 100 mg et 180 mg de Milnacipran peuvent être mises en gélule de tailles n° 1 ou n° 0+.

De plus, ces formules conditionnées en blister PVC (250 µm)/PVDC (40 g/m²), aluminium (20 µm) sont stables pendant au moins 6 mois à 30°C/70 % HR.

### Exemple n° 15 :

Cet exemple se différencie du précédent par le fait que l'opération d'enrobage des minigranules se fait sur un appareillage semi-industriel de type GLATT GPCG120.

Les proportions des différents composants sont multipliées par le facteur 3,6 par rapport à l'exemple n° 14 et par un facteur 54 par rapport à l'exemple 13.

Les différents paramètres opératoires sont adaptés en conséquence.

En fonction de la qualité de liquide d'enrobage déposée sur les minigranules, le taux de polymère sec d'enrobage peut représenter 7,5 et 10 % du poids de minigranules non enrobés.

Description des formules :

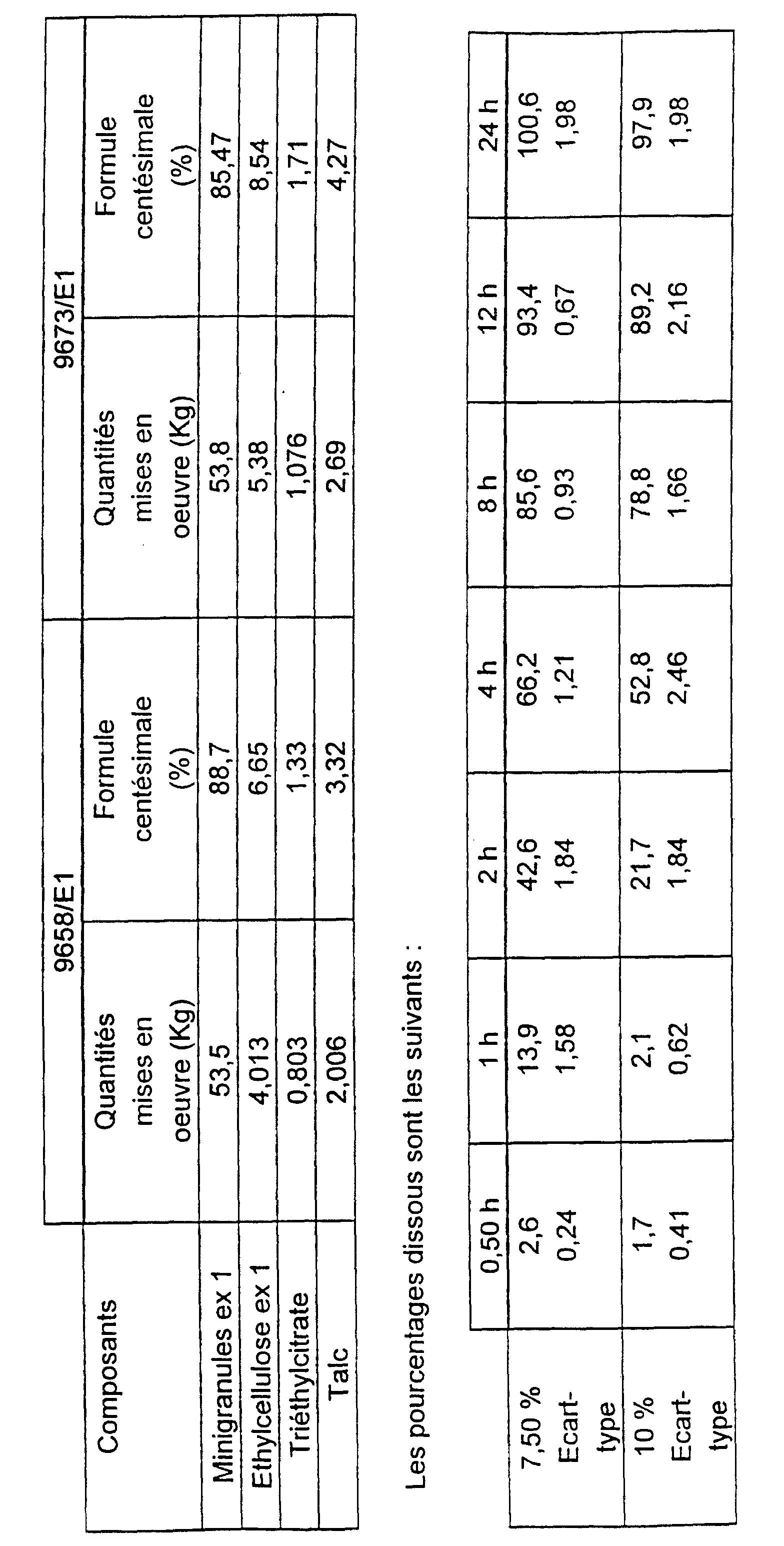

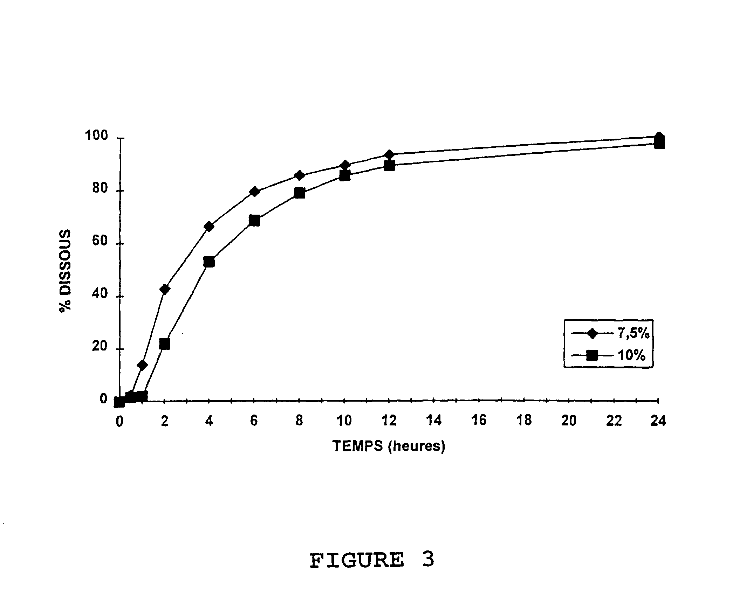

De façon surprenante, les lots 9658/E1 et 9673/E1 renfermant une concentration en Milnacipran par minigranule non enrobé de 510 µg atteignent l'objectif in vitro évoqué auparavant. Les courbes de cinétiques de dissolution moyennes correspondantes sont donnée dans la figure 3 annexée.

Ces formules aux doses unitaires de 60 à 240 mg, et plus particulièrement de 60, 120 et 240 mg de Milnacipran, peuvent être mises en gélule de tailles n° 3, n° 1 ou n° 00.

### Exemple n° 16 :

L'exemple n° 16 se différencie de l'ensemble des exemples précédents par le fait que les microgranules actifs sont réalisés suivant l'exemple n° 3, c'est à dire par extrusion axiale puis sphéronisation. La préparation de la solution d'enrobage ainsi que l'opération d'enrobage se font suivant l'exemple précédent.

**Description des formules :**

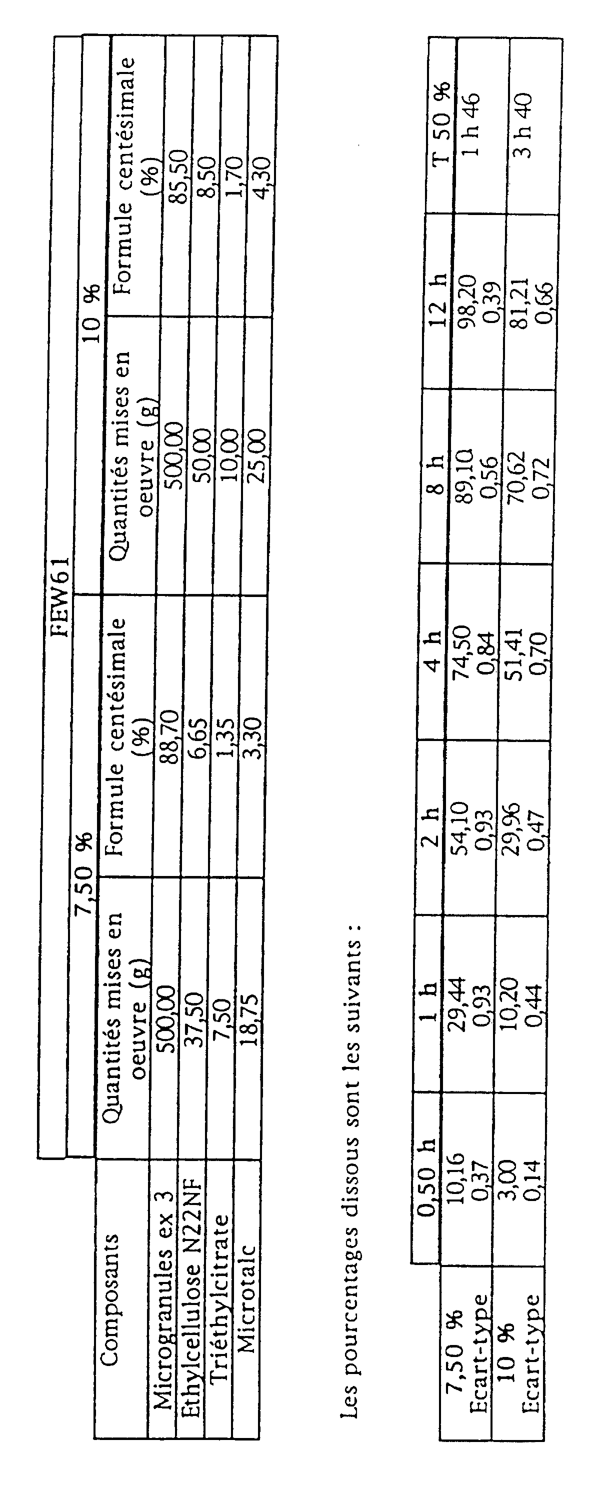

De façon surprenante, la formule FEW61 à 7,5 % et 10 % de polymère, renfermant une concentration de Milnacipran par minigranule de 500 µg, dont les épaisseurs de film sont égales à 21 et 28 µm (masses surfaciques égales à 1,25 et 1,67 mg/cm²) atteignent l'objectif in vitro évoqué auparavant.

Ces formules aux doses unitaires de 60 à 240 mg, et plus particulièrement 60 mg et 180 mg de Milnacipran, peuvent être mises en gélule de tailles n° 3 et n° 0+.

### Exemple n° 17 :

Cet exemple se différencie des exemples précédents par le fait que les microgranules actifs ont été réalisés suivant l'exemple n° 5, c'est-à-dire par rotogranulation en lit d'air fluidisé.

La préparation de la dispersion d'enrobage ainsi que l'opération d'enrobage se fait suivant l'exemple n° 10, sachant que dans ce cas il s'agit de Surelease E-7-7060 dont le plastifiant est le Miglyol et non pas le Dibuthyl sébaçate.

En fonction de la quantité de liquide d'enrobage déposée sur les minigranules, le taux de polymère sec d'enrobage peut représenter 15 et 20 % du poids des minigranules non enrobés.

**Description des formules :**

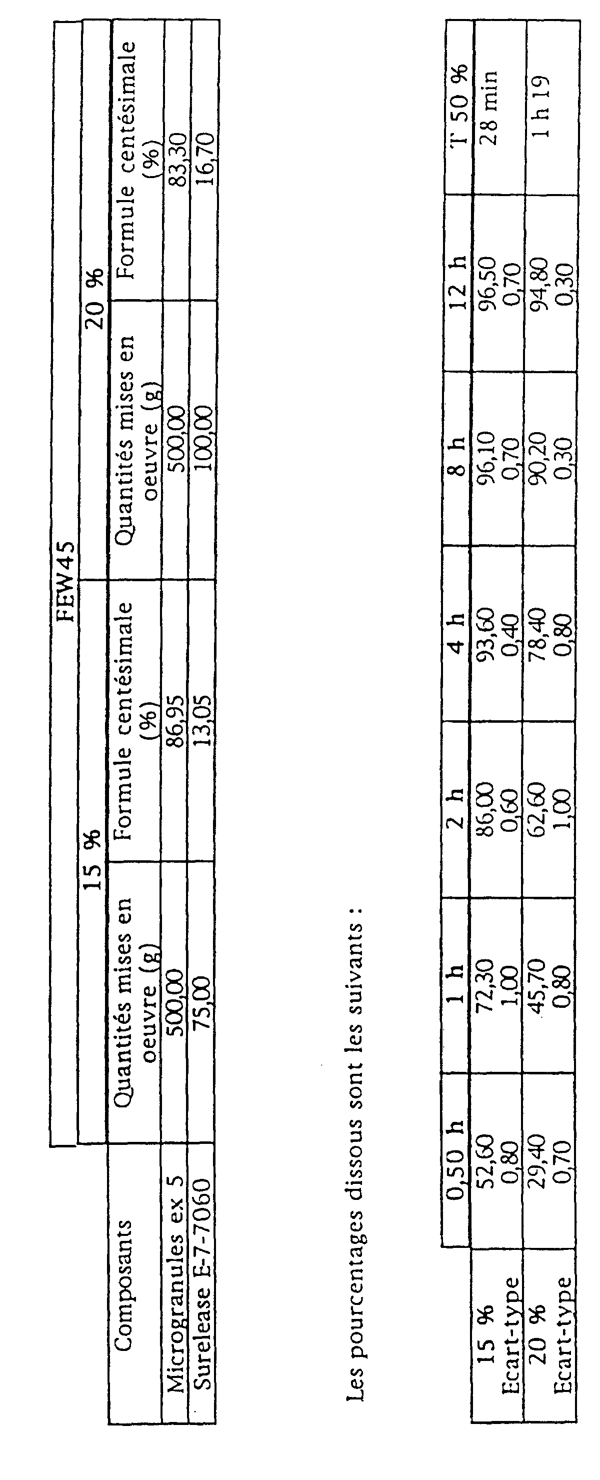

Les formules à 15 % et à 20 % de polymère, dont la concentration de Milnacipran par minigranule est de 500 µg et dont les épaisseurs de film sont égales à 65 µm et 87,5 µm (masse surfacique égale à 3,13 et 4,2 mg/cm²), ne permettent pas d'atteindre l'objectif in vitro précédemment cité.

## Revendications

1. Forme galénique à libération prolongée, destinée à l'administration par voie orale en une prise journalière unique de 60 à 240 mg de Milnacipran, se présentant sous une forme multiparticulaire réunissant une pluralité de minigranules comportant chacune une minisphère active comprenant un noyau de saccharose et/ou d'amidon de granulométrie comprise entre 200 et 2 000 µm et renfermant de 150 à 1 000 µg de Milnacipran ainsi qu'un agent liant, chaque minigranule étant enrobé par un film, à base d'au moins un polymère non hydrosoluble mais perméable aux liquides physiologiques, d'épaisseur comprise entre 20 et 100 µm, ladite forme galénique permettant une libération in vitro correspondant au profil suivant :
* entre 10 et 55 % de la dose libérée en 2 heures
* entre 40 et 75 % de la dose libérée en 4 heures
* entre 70 et 90 % de la dose libérée en 8 heures
* entre 80 et 100 % de la dose libérée en 12 heures

2. Forme galénique selon la revendication 1, **caractérisée en ce que** la dose unitaire de Milnacipran administrable est plus particulièrement comprise entre 60 et 240 mg et plus particulièrement 120 mg.

3. Forme galénique selon l'une des revendications 1 ou 2, **caractérisée en ce que** le Milnacipran est utilisé sous la forme d'une dose thérapeutiquement équivalente de son énantiomère Cis-D.

4. Forme galénique selon l'une des revendications 1 à 3, **caractérisée en ce que** les minisphères actives non enrobées, fabriquées dans une turbine ou dans un lit d'air fluidisé, sont susceptibles d'être obtenues à partir de non-pareils de taille comprise entre 200 et 2 000 µm, plus précisément entre 500 et 1 000 µm.

5. Forme galénique selon la revendication 4, **caractérisée en ce que** les non-pareils sont composés de saccharose et/ou d'amidon, de préférence d'un mélange d'environ 75 % saccharose et d'environ 25 % d'amidon.

6. Forme galénique selon l'une des revendications 1 à 3 **caractérisée en ce que** les minigranules actifs non enrobés sont susceptibles d'être obtenus par extrusion-sphéronisation.

7. Forme galénique selon la revendication 6, **caractérisée en ce que**, au cours de l'opération d'extrusion-sphéronisation, on utilise un agent liant choisi parmi la cellulose microcristalline, l'hydroxypropyl cellulose, la carboxyméthyl cellulose, la gélatine, le lactose, l'amidon, un mono- ou di- ou tri-glycéride, les gommes guar, arabique, adragante, les pectines, les alginates et leurs mélanges.

8. Forme galénique selon la revendication 7, **caractérisée en ce que** l'agent liant utilisé est la cellulose microcristalline dans des proportions massiques comprises entre 25 et 75 %, plus précisément égale à 50 %.

9. Forme galénique selon l'une des revendications 6 à 8, **caractérisée en ce que**, au cours de l'opération d'extrusion-sphéronisation, on utilise un solvant de mouillage choisi parmi l'eau purifiée, l'éthanol, l'isopropanol et leurs mélanges respectifs.

10. Forme galénique selon la revendication 4, **caractérisée en ce que** l'agent liant utilisé est la PVP de poids moléculaire voisin de 50 000 Da en solution dans l'éthanol, CH₂Cl₂, l'acétone, l'isopropanol et leurs mélanges, la masse de PVP représentant 3 à 50 %, de préférence environ 20 % de celle de la solution de mouillage et de préférence 2 à 4 % de celle des minisphères actives non enrobées.

11. Forme galénique selon la revendication 10, **caractérisée en ce que** les minisphères contiennent en outre un agent anti-adhérent, tel que le talc, à raison de 0,5 à 20 %, de préférence 3 à 4 % en poids par rapport au poids des microsphères actives non enrobées.

12. Forme galénique selon la revendication 4, **caractérisée en ce que** l'agent liant utilisé est choisi parmi l'hydroxypropylméthyl cellulose, la méthyl cellulose ou l'hydroxypropyl cellulose, la carboxyméthyl cellulose, la gélatine, les gommes arabique, adragante, guar, les pectines, les alginates, une solution de saccharose, et leurs mélanges.

13. Forme galénique selon l'une des revendications 1 à 4 et 10 à 12, **caractérisée en ce que** le Milnacipran en dispersion dans l'isopropanol à un taux massique de 10 à 40 %, préférentiellement 20 %, est pulvérisé sur les non-pareils à un taux massique de 40 à 50 %, plus précisément 46 %.

14. Forme galénique selon la revendication 13, **caractérisée en ce que** en utilisant des non-pareils de taille comprise entre 710 et 850 µm, on obtient des minisphères actives non enrobées contenant 440 à 555 µg de Milnacipran.

15. Forme galénique selon la revendication 13, **caractérisée en ce que** en utilisant des non-pareils de taille comprise entre 850 et 1 000 µm, on obtient des minisphères actives non enrobées contenant 680 à 850 µg de Milnacipran.

16. Forme galénique selon la revendication 13, **caractérisée en ce que** en utilisant des non-pareils de taille comprise entre 500 et 600 µm, on obtient des minisphères actives non enrobées contenant 150 à 185 µg, plus précisément 170 µg de Milnacipran.

17. Forme galénique selon l'une des revendications 1 à 16, **caractérisée en ce que** le film d'enrobage des minigranules est susceptible d'être obtenu à l'aide d'un agent d'enrobage constitué d'un ou plusieurs copolymères méthacryliques de type poly (éthacrylate, méthyl méthacrylate) en dispersion aqueuse ou de type poly (éthyl acrylate, méthyl méthacrylate, chlorure de triméthyl ammonium, méthyl méthacrylate) en solvant organique ou en dispersion aqueuse, la masse de polymère sec utilisé étant de préférence comprise entre 5 et 50 % de celle des minigranules.

18. Forme galénique selon la revendication 16, **caractérisée en ce que** l'agent d'enrobage est constitué d'alkyl-cellulose en solution aqueuse dont l'extrait sec représente 5 à 30 % du poids des minigranules.

19. Forme galénique selon la revendication 16, **caractérisée en ce que** l'agent d'enrobage est constitué d'alkyl-cellulose en solution dans un solvant ou un mélange de solvants organiques, dont l'extrait sec représente 2,5 à 50 % du poids des minigranules.

20. Forme galénique selon la revendication 17, **caractérisée en ce que** l'agent d'enrobage contient un agent anti-adhérent choisi parmi le talc, les oxydes métalliques ou des silices.

21. Forme galénique selon l'une des revendications 17 à 20, **caractérisée en ce que** l'agent d'enrobage contient un plastifiant est choisi parmi le triéthylcitrate, le dibutyl sébaçate, le dibutylphtalate, diéthylphtalate, l'acétyl triéthylcitrate, le tributylcitrate, l'acétyl triéthylcitrate, la triacétine, les polyéthylènes glycols, le propylène glycol, les glycérols, l'huile de coco fractionnée et l'huile de ricin.

22. Forme galénique selon l'une des revendications 1 à 16, pour laquelle l'opération d'enrobage réalisée en lit d'air fluidisé fait intervenir un agent d'enrobage de type éthylcellulose en solution éthanolique à un taux de polymère sec par rapport au poids des minisphères compris entre 3 et 15 % et de préférence compris entre 4 et 12,5 %, conduisant à une épaisseur de film comprise entre 20 et 80 µm.

23. Forme galénique selon la revendication 22, **caractérisée par le fait que** le film d'enrobage à base d'éthylcellulose comprend un plastifiant, le triéthylcitrate ou le dibutyl sébaçate, à un taux exprimé par rapport au poids de polymère sec compris entre 15 et 25 % et de préférence égal à environ 20 %.

24. Forme galénique selon la revendication 23, **caractérisée par le fait que** le film d'enrobage à base d'éthylcellulose comprend un agent de charge tel que le talc, dont le taux exprimé par rapport au poids de polymère sec est compris entre 20 et 70 % et de préférence égal à environ 50 %.

25. Forme galénique selon l'une des revendications 1 à 16, pour laquelle l'opération de pelliculage réalisée en lit d'air fluidisé fait appel à un agent d'enrobage de type éthylcellulose en suspension aqueuse, à un taux de polymère sec par rapport au poids des minisphères compris entre 5 et 30 % de préférence entre 12,5 % et 17,5 % conduisant à une épaisseur de film compris entre 60 et 90 µm.

26. Forme galénique selon l'une des revendications 1 à 16, pour laquelle l'opération d'enrobage réalisée en lit d'air fluidisé comprend un agent d'enrobage de type polyéthylacrylate méthylméthacrylate en dispersion aqueuse, à un taux de polymère sec par rapport au poids des minisphères compris entre 10 et 20 % et plus particulièrement entre 12,5 % et 15 % soit une épaisseur de film comprise entre 60 et 80 µm.

27. Forme galénique selon l'une des revendications 25 et 26, **caractérisée par le fait que** le film d'enrobage comprend un agent de charge, en particulier le talc, à un taux exprimé par rapport au poids sec de l'agent d'enrobage polymère compris entre 20 et 70 %, de préférence égal à environ 50 %.

## Patentansprüche

1. Galenische Form mit protrahierter Freisetzung zur oralen Verabreichung von 60 bis 240 mg Milnacipran in einer einzigen Tagesdosis in multipartikulärer Form mit einer Kombination aus mehreren Minikörnern, die jeweils eine aktive Minikugel mit einem Kern aus Saccharose und/oder Stärke mit einer Korngröße zwischen 200 und 2000 µm aufweisen und 150 bis 1000 µg Milnacipran sowie ein Bindemittel enthalten, wobei jedes Minikom mit einem Film mit einer Dicke zwischen 20 und 100 µm auf der Basis wenigstens eines wasserunlöslichen, aber für physiologische Flüssigkeiten durchlässigen Polymers überzogen ist, wobei die galenische Form eine in-vitro-Freisetzung gemäß folgendem Profil gestattet:
* Freisetzung von 10 bis 55% der Dosis in 2 Stunden
* Freisetzung von 40 bis 75% der Dosis in 4 Stunden
* Freisetzung von 70 bis 90% der Dosis in 8 Stunden
* Freisetzung von 80 bis 100% der Dosis in 12 Stunden.

2. Galenische Form nach Anspruch 1, **dadurch gekennzeichnet, daß** die verabreichbare Milnacipran-Einzeldosis insbesondere zwischen 60 und 240 mg liegt und insbesondere 120 mg beträgt.

3. Galenische Form nach Anspruch 1 oder 2, **dadurch gekennzeichnet, daß** das Milnacipran in Form einer therapeutisch äquivalenten Dosis seines cis-D-Enantiomers verwendet wird.

4. Galenische Form nach einem der Ansprüche 1 bis 3, **dadurch gekennzeichnet, daß** die unbeschichteten, in einer Turbine oder einer Luft-Wirbelschicht hergestellten aktiven Minikugeln aus Nonpareils mit einer Größe zwischen 200 und 2000 µm, insbesondere zwischen 500 und 1000 µm, erhältlich sind.

5. Galenische Form nach Anspruch 4, **dadurch gekennzeichnet, daß** die Nonpareils aus Saccharose und/oder Stärke und vorzugsweise aus einem Gemisch aus etwa 75% Saccharose und etwa 25% Stärke zusammengesetzt sind.

6. Galenische Form nach einem der Ansprüche 1 bis 3, **dadurch gekennzeichnet, daß** die unbeschichteten aktiven Minikörner durch Extrusions-Sphäroidisierung erhältlich sind.

7. Galenische Form nach Anspruch 6, **dadurch gekennzeichnet, daß** beim Vorgang der Extrusions-Sphärodisierung ein unter mikrokristalliner Cellulose, Hydroxypropylcellulose, Carboxymethylcellulose, Gelatine, Lactose, Stärke, einem Mono-, Di- oder Triglycerid, Guar-Gummi, Gummi arabicum, Tragant, Pektinen, Alginaten und Gemischen davon ausgewähltes Bindemittel verwendet wird.

8. Galenische Form nach Anspruch 7, **dadurch gekennzeichnet, daß** als Bindemittel mikrokristalline Cellulose in Massenanteilen zwischen 25 und 75% und insbesondere in einem Massenanteil von 50% verwendet wird.

9. Galenische Form nach einem der Ansprüche 6 bis 8, **dadurch gekennzeichnet, daß** beim Vorgang der Extrusions-Sphärodisierung ein unter gereinigtem Wasser, Ethanol, Isopropanol und deren jeweiligen Gemischen ausgewähltes Benetzungslösungsmittel verwendet wird.

10. Galenische Form nach Anspruch 4, **dadurch gekennzeichnet, daß** als Bindemittel PVP mit einem Molekulargewicht nahe 50.000 Da in Lösung in Ethanol, CH₂Cl₂, Aceton, Isopropanol und Gemischen davon verwendet wird, wobei sich die Masse des PVP auf 3 bis 50% und vorzugsweise ungefähr 20% der Masse der Benetzungslösung und vorzugsweise auf 2 bis 4% der **Masse der unbeschichteten aktiven Minikugeln beläuft.**

11. Galenische Form nach Anspruch 10, **dadurch gekennzeichnet, daß** die Minikugeln außerdem auch noch ein Antihaftmittel, wie Talk, in einer Menge von 0,5 bis 20 Gew.-%, vorzugsweise 3 bis 4 Gew.-%, bezogen auf das Gewicht der unbeschichteten aktiven Mikrokugeln, enthalten.

12. Galenische Form nach Anspruch 4, **dadurch gekennzeichnet, daß** das verwendete Bindemittel unter Hydroxypropylmethylcellulose, Methylcellulose oder Hydroxypropylcellulose, Carboxymethylcellulose, Gelatine, Gummi arabicum, Tragant, Guar-Gummi, Pektinen, Alginaten, einer Saccharoselösung und Gemischen davon ausgewählt wird.

13. Galenische Form nach einem der Ansprüche 1 bis 4 und 10 bis 12, **dadurch gekennzeichnet, daß** das Milnacipran als Dispersion in Isopropanol in einem Massenanteil von 10 bis 40%, vorzugsweise 20%, in einem Massenanteil von 40 bis 50%, insbesondere 46%, auf die Nonpareils gesprüht wird.

14. Galenische Form nach Anspruch 13, **dadurch gekennzeichnet, daß** unter Verwendung von Nonpareils mit einer Größe zwischen 710 und 850 µm unbeschichtete aktive Minikugeln mit 440 bis 555 µg Milnacipran erhalten werden.

15. Galenische Form nach Anspruch 13, **dadurch gekennzeichnet, daß** unter Verwendung von Nonpareils mit einer Größe zwischen 850 und 1000 µm unbeschichtete aktive Minikugeln mit 680 bis 850 µg Milnacipran erhalten werden.

16. Galenische Form nach Anspruch 13, **dadurch gekennzeichnet, daß** unter Verwendung von Nonpareils mit einer Größe zwischen 500 und 600 µm unbeschichtete aktive Minikugeln mit 150 bis 185 µg, insbesondere 170 µg, Milnacipran erhalten werden.

17. Galenische Form nach einem der Ansprüche 1 bis 16, **dadurch gekennzeichnet, daß** der Überzugsfilm für die Minikörner mit Hilfe eines Beschichtungsmittels erhältlich ist, das aus einem oder mehreren Methacryl-Copolymeren des Typs Poly(ethylacrylat-methylmethacrylat) in wäßriger Dispersion oder des Typs Poly(ethylacrylat-methylmethacrylattrimethylammoniumchlorid-methylmethacrylat) in einem organischen Lösungsmittel oder wäßriger Dispersion zusammengesetzt ist, wobei die verwendete Masse an trockenem Polymer vorzugsweise zwischen 5 und 50% der Masse der Minikörner liegt.

18. Galenische Form nach Anspruch 16, **dadurch gekennzeichnet, daß** das Beschichtungsmittel aus Alkylcellulose in wäßriger Lösung, deren Feststoffgehalt sich auf 5 bis 30 Gew.-%, bezogen auf die Minikörner, beläuft, zusammengesetzt ist.

19. Galenische Form nach Anspruch 16, **dadurch gekennzeichnet, daß** das Beschichtungsmittel aus Alkylcellulose in Lösung in einem organischen Lösungsmittel oder Gemisch von organischen Lösungsmitteln, deren Feststoffgehalt sich auf 2,5 bis 50 Gew.-%, bezogen auf die Minikörner, beläuft, zusammengesetzt ist.

20. Galenische Form nach Anspruch 17, **dadurch gekennzeichnet, daß** das Beschichtungsmittel ein unter Talk, Metalloxiden oder Siliciumoxiden ausgewähltes Antihaftmittel enthält.

21. Galenische Form nach einem der Ansprüche 17 bis 20, **dadurch gekennzeichnet, daß** das Beschichtungsmittel einen unter Triethylcitrat, Dibutylsebacat, Dibutylphthalat, Diethylphthalat, Acetyltriethylcitrat, Tributylcitrat, Triacetin, Polyethylen-glykolen, Propylenglykol, Glycerinen, fraktioniertem Kokosnußöl und Rizinusöl ausgewählten Weichmacher enthält.

22. Galenische Form nach einem der Ansprüche 1 bis 16, für die bei der in einer Luft-Wirbelschicht erfolgenden Beschichtung ein Beschichtungsmittel vom Typ Ethylcellulose in ethanolischer Lösung mit einem Gehalt an trockenem Polymer zwischen 3 und 15% und vorzugsweise zwischen 4 und 12,5%, bezogen auf das Gewicht der Minikugeln, verwendet wird, was eine Filmdicke zwischen 20 und 80 µm ergibt.

23. Galenische Form nach Anspruch 22, **dadurch gekennzeichnet, daß** der auf Ethylcellulose basierende Überzugsfilm als Weichmacher Triethylcitrat oder Dibutylsebacat in einer Menge zwischen 15 und 25% und vorzugsweise gleich ungefähr 20%, bezogen auf das Gewicht an trockenem Polymer, enthält.

24. Galenische Form nach Anspruch 23, **dadurch gekennzeichnet, daß** der auf Ethylcellulose basierende Überzugsfilm einen Füllstoff, wie Talk, in einer Menge zwischen 20 und 70% und vorzugsweise gleich ungefähr 50%, bezogen auf das Gewicht an trockenem Polymer, enthält.

25. Galenische Form nach einem der Ansprüche 1 bis 16, für die bei der in einer Luft-Wirbelschicht erfolgenden Filmbeschichtung ein Beschichtungsmittel vom Typ Ethylcellulose in wäßriger Suspension mit einem Gehalt an trockenem Polymer zwischen 5 und 30% und vorzugsweise zwischen 12,5 und 17,5%, bezogen auf das Gewicht der Minikugeln, verwendet wird, was eine Filmdicke zwischen 60 und 90 µm ergibt.

26. Galenische Form nach einem der Ansprüche 1 bis 16, für die bei der in einer Luft-Wirbelschicht erfolgenden Beschichtung ein Beschichtungsmittel vom Typ Poly(ethylacrylat-methylmethacrylat) in wäßriger Dispersion mit einem Gehalt an trockenem Polymer zwischen 10 und 20% und vorzugsweise zwischen 12,5 und 15%, bezogen auf das Gewicht der Minikugeln, verwendet wird, was eine Filmdicke zwischen 60 und 80 µm ergibt.

27. Galenische Form nach Anspruch 25 und 26, **dadurch gekennzeichnet, daß** der Überzugsfilm einen Füllstoff, insbesondere Talk, in einer Menge zwischen 20 und 70% und vorzugsweise gleich ungefähr 50%, bezogen auf das Trockengewicht des polymeren Beschichtungsmittels, enthält.

## Claims

1. Prolonged-release galenic form, intended for oral administration in a single daily dose of 60 to 240 mg of Milnacipran, provided in a multiparticulate form combining a plurality of minigranules each containing an active minisphere comprising a sucrose and/or starch core having a particle size of between 200 and 2000 µm and containing 150 to 1000 µg of Milnacipran as well as a binding agent, each minigranule being coated with a film, based on at least one polymer insoluble in water but permeable to physiological fluids, having a thickness of between 20 and 100 µm, said galenic form allowing an in-vitro release corresponding to the following pattern:
* between 10 and 55% of the dose released in 2 hours,
* between 40 and 75% of the dose released in 4 hours,
* between 70 and 90% of the dose released in 8 hours,
* between 80 and 100% of the dose released in 12 hours.

2. Galenic form according to Claim 1, **characterized in that** the administrable Milnacipran unit dose is more particularly between 60 and 240 mg and more particularly 120 mg.

3. Galenic form according to either of Claims 1 and 2, **characterized in that** the Milnacipran is used in the form of a therapeutically equivalent dose of its Cis-D enantiomer.

4. Galenic form according to one of Claims 1 to 3, **characterized in that** the uncoated active minispheres, manufactured in a pan or in a fluidized air bed, are capable of being obtained from nonpareils having a size of between 200 and 2000 µm, more precisely between 500 and 1000 µm.

5. Galenic form according to Claim 4, **characterized in that** the nonpareils are composed of sucrose and/or starch, preferably a mixture of about 75% sucrose and about 25% starch.

6. Galenic form according to one of Claims 1 to 3, **characterized in that** the uncoated active minigranules are capable of being obtained by extrusionspheronization.

7. Galenic form according to Claim 6, **characterized in that**, during the extrusionspheronization operation, a binding agent is used which is chosen from microcrystalline cellulose, hydroxypropylcellulose, carboxymethylcellulose, gelatin, lactose, starch, a mono- or di- or triglyceride, guar gum, gum arabic, gum tragacanth, pectins, alginates and mixtures thereof.

8. Galenic form according to Claim 7, **characterized in that** the binding agent used is microcrystalline cellulose in proportions by mass of between 25 and 75%, more precisely equal to 50%.

9. Galenic form according to one of Claims 6 to 8, **characterized in that**, during the extrusionspheronization operation, a wetting solvent is used which is chosen from purified water, ethanol, isopropanol and their respective mixtures.

10. Galenic form according to Claim 4, **characterized in that** the binding agent used is PVP having a molecular weight of close to 50,000 Da in solution in ethanol, CH₂Cl₂, acetone, isopropanol and mixtures thereof, the mass of PVP representing 3 to 50%, preferably about 20% of that of the wetting solution and preferably 2 to 4% of that of the uncoated active minispheres.

11. Galenic form according to Claim 10, **characterized in that** the minispheres contain, in addition, an antiadhering agent such as talc in an amount of 0.5 to 20%, preferably 3 to 4% by weight relative to the weight of the uncoated active microspheres.

12. Galenic form according to Claim 4, **characterized in that** the binding agent used is chosen from hydroxypropylmethylcellulose, methyl cellulose or hydroxypropylcellulose, carboxymethylcellulose, gelatin, gum arabic, gum tragacanth, guar gum, pectins, alginates, a solution of sucrose, and mixtures thereof.

13. Galenic form according to one of Claims 1 to 4 and 10 to 12, **characterized in that** the Milnacipran in dispersion in isopropanol in an amount by mass of 10 to 40%, preferably 20%, is sprayed on the nonpareils in an amount by mass of 40 to 50%, more precisely 46%.

14. Galenic form according to Claim 13, **characterized in that** by using nonpareils having a size of between 710 and 850 µm, uncoated active minispheres containing 440 to 555 µg of Milnacipran are obtained.

15. Galenic form according to Claim 13, **characterized in that** by using nonpareils having a size of between 850 and 1000 µm, uncoated active minispheres containing 680 to 850 µg of Milnacipran are obtained.

16. Galenic form according to Claim 13, **characterized in that** by using nonpareils having a size of between 500 and 600 µm, uncoated active minispheres containing 150 to 185 µg, more precisely 170 µg of Milnacipran, are obtained.

17. Galenic form according to one of Claims 1 to 16, **characterized in that** the film for coating the minigranules is capable of being obtained with the aid of a coating agent consisting of one or more methacrylic copolymers of the poly(ethacrylate, methyl methacrylate) type in aqueous dispersion or of the poly(ethyl acrylate, methyl methacrylate, trimethylammonium chloride, methyl methacrylate) type in an organic solvent or in an aqueous dispersion, the mass of dry polymer used being preferably between 5 and 50% of that of the minigranules.

18. Galenic form according to Claim 16, **characterized in that** the coating agent consists of alkyl cellulose in aqueous solution whose dry extract represents 5 to 30% of the weight of the minigranules.

19. Galenic form according to Claim 16, **characterized in that** the coating agent consists of alkyl cellulose in solution in a solvent or a mixture of organic solvents, whose dry extract represents 2.5 to 50% of the weight of the minigranules.

20. Galenic form according to Claim 17, **characterized in that** the coating agent contains an antiadhering agent chosen from talc, metal oxides or silicas.

21. Galenic form according to one of Claims 17 to 20, **characterized in that** the coating agent contains a plasticizer, is chosen from triethyl citrate, dibutyl sebacate, dibutyl phthalate, diethyl phthalate, acetyl triethyl citrate, tributyl citrate, acetyl triethyl citrate, triacetin, polyethylene glycols, propylene glycol, glycerols, fractionated coconut oil and castor oil.

22. Galenic form according to one of Claims 1 to 16, for which the coating operation carried out in a fluidized air bed involves a coating agent of the ethyl cellulose type in ethanolic solution at a dry polymer level relative to the weight of the minispheres of between 3 and 15% and preferably between 4 and 12.5%, leading to a film thickness of between 20 and 80 µm.

23. Galenic form according to Claim 22, **characterized in that** the coating film based on ethyl cellulose comprises a plasticizer, triethyl citrate or dibutyl sebacate, in an amount expressed relative to the dry polymer weight of between 15 and 25% and preferably equal to about 20%.

24. Galenic form according to Claim 23, **characterized in that** the coating film based on ethyl cellulose comprises a filling agent such as talc whose amount expressed relative to the dry polymer weight is between 20 and 70% and preferably equal to about 50%.

25. Galenic form according to one of Claims 1 to 16, for which the film-coating operation carried out in a fluidized air bed involves a coating agent of the ethyl cellulose type in aqueous suspension, at a dry polymer level relative to the weight of the minispheres of between 5 and 30%, preferably between 12.5% and 17.5%, leading to a film thickness of between 60 and 90 µm.

26. Galenic form according to one of Claims 1 to 16, for which the coating operation carried out in a fluidized air bed comprises a coating agent of the polyethyl acrylate methyl methacrylate type in aqueous dispersion, at a dry polymer level relative to the weight of the minispheres of between 10 and 20% and more particularly between 12.5% and 15%, that is to say a film thickness of between 60 and 80 µm.

27. Galenic form according to either of Claims 25 and 26, **characterized in that** the coating film comprises a filling agent, in particular talc, at a level expressed relative to the dry weight of the polymer coating agent of between 20 and 70%, preferably equal to about 50%.
